# EUROPÄISCHE PATENTSCHRIFT

(11) **EP 0 699 663 B1**
(45) Veröffentlichungstag und Bekanntmachung des Hinweises auf die Patenterteilung: **04.11.1998**
(21) Anmeldenummer: 95113104.4
(22) Anmeldetag: 21.08.1995
(51) Int. Cl.: C07C 317/44, C07C 317/46, C07C 311/16, C07C 311/29, C07C 279/22, A61K 31/155, A61K 31/18, A61K 31/275

(54) **Alkyl-benzoylguanidin-Derivate**
Alkyl-benzoylguanidine derivatives
Dérivés d'alkyl-benzoylguanidine

(30) Priorität: 31.08.1994 DE 4430916
(43) Veröffentlichungstag der Anmeldung: 06.03.1996
(73) Patentinhaber: MERCK PATENT GmbH, 64293 Darmstadt (DE)
(72) Erfinder: Gericke, Rolf, Dr., D-64342 Seeheim (DE); Baumgarth, Manfred, Dr., D-64297 Darmstadt (DE); Dorsch, Dieter, Dr., D-64372 Ober-Ramstadt (DE); Minck, Klaus-Otto, Dr., D-64372 Ober Ramstadt (DE); Beier, Norbert, Dr., D-64354 Reimheim (DE)

(56) Entgegenhaltungen:
- EP-A- 0 416 499
- EP-A- 0 556 672
- EP-A- 0 556 673
- EP-A- 0 556 674
- EP-A- 0 577 024
- EP-A- 0 589 336
- EP-A- 0 603 650
- EP-A- 0 640 588

## Beschreibung

Die Erfindung betrifft ortho-substituierte Alkyl-benzoylguanidin-Derivate der Formel I 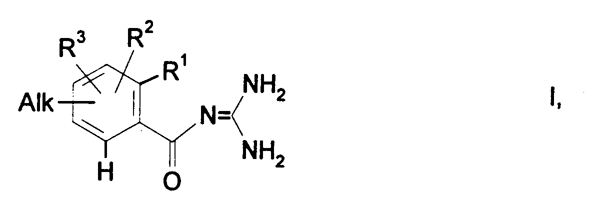 worin
- R¹: A, CF₃, CH₂F, CHF₂, C₂F₅, CN, NO₂, Hal, C≡CH oder -X-R⁴,
- R² und R³: jeweils unabhängig voneinander H, Hal, A, -X-R⁴, CN, NO₂, CF₃, CH₂F, CHF₂, C₂F₅, CH₂CF₃, -SOₙ-R⁶, -SO₂NR⁴R⁵, Ph, OPh oder Het,
- R⁴: H, A, Cycloalkyl mit 5 bis 7 C-Atomen, Cycloalkylmethyl mit 6 bis 8 C-Atomen, CF₃, CH₂F, CHF₂, CH₂CF₃, Ph oder -CH₂-Ph,
- R⁵: H oder A oder aber
- R⁴ und R⁵: zusammen auch Alkylen mit 4 bis 5 C-Atomen, wobei eine CH₂-Gruppe auch durch O, S, NH, N-A oder N-CH₂-Ph ersetzt sein kann,
- R⁶: A oder Ph,
- Alk: einen geradkettigen oder verzweigten C₁-C₈-Alkyl-Rest, C₃-C₈-Cycloalkyl, welcher unsubstituiert oder durch A ein-, zwei- oder dreifach substituiert sein kann, -CR⁷=CHR^{7'}oder -C≡CR⁷,
- R⁷ und R^{7'}: jeweils unabhängig voneinander H, A, Ph oder Het,
- Het: einen ein- oder zweikernigen gesättigten, ungesättigten oder aromatischen Heterocyclus mit 1 bis 4 N-, O-, und/oder S-Atomen, über N oder C gebunden, der unsubstituiert oder ein-, zwei- oder dreifach durch Hal, CF₃, A, -X-R⁴, CN, NO₂ und/oder Carbonylsauerstoff substituiert sein kann,
- A: Alkyl mit 1 bis 6 C-Atomen,
- Hal: F, Cl, Br oder I,
- X: O oder S,
- Ph: unsubstituiertes oder ein-, zwei- oder dreifach durch A, OA, NR⁴R⁵, F, Cl, Br, I oder CF₃ substituiertes Phenyl und
- n: 1 oder 2,
bedeuten,
sowie deren physiologisch unbedenkliche Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen.

Bei den neuen Verbindungen handelt es sich um Inhibitoren des zellulären Na⁺/H⁺-Antiporters, d.h. um Wirkstoffe, die den Na⁺/H⁺-Austauschmechanismus der Zellen hemmen (Düsing et al., Med. Klin. 87, 378-384 (1992)) und die somit gute Antiarrhythmika darstellen, die sich insbesondere zur Behandlung von Arrhythmien eignen, die als Folge von Sauerstoffmangel auftreten.

Der bekannteste Wirkstoff der Gruppe der Acylguanidine ist Amilorid. Diese Substanz zeigt jedoch in erster Linie eine blutdrucksenkende und saluretische Wirkung, was insbesondere bei der Behandlung von Herz-Rhythmus-Störungen unerwünscht ist, während die antiarrhythmischen Eigenschaften nur sehr schwach ausgeprägt sind.

Darüber hinaus kennt man strukturell ähnliche Verbindungen beispielsweise aus EP 04 16 499 und EP 06 03 650.

Gegenstand der Erfindung sind Verbindungen der Formel I sowie ihre physiologisch unbedenklichen Salze.

Die erfindungsgemäßen Substanzen der vorliegenden Anmeldung zeigen eine gute kardioprotektive Wirkung und eignen sich daher besonders zur Infarktbehandlung, Infarktprophylaxe und zur Behandlung von Angina pectoris. Ferner wirken die Substanzen allen pathologischen hypoxischen und ischämischen Schädigungen entgegen, so daß die dadurch primär oder sekundär verursachten Krankheiten behandelt werden können. Die Wirkstoffe sind ebenfalls für präventive Anwendungen gut geeignet.

Aufgrund der protektiven Wirkungen dieser Substanzen bei pathologischen hypoxischen oder ischämischen Situationen resultieren daraus weitere Anwendungsmöglichkeiten bei chirurgischen Eingriffen zum Schutz zeitweilig minderversorgter Organe, bei Organtransplantationen zum Schutz der entnommenen Organe, bei angioplastischen Gefäß- oder Herzeingriffen, bei Ischämien des Nervensystems, bei der Therapie von Schockzuständen und zur präventiven Verhinderung der essentiellen Hypertonie.

Ferner können die Verbindungen auch als Therapeutika bei durch Zellproliferation bedingten Erkrankungen wie Arteriosklerose, diabetische Spätkomplikationen, Tumorerkrankungen, fibrotischen Erkrankungen, insbesondere von Lunge, Leber und Nieren sowie Organhypertrophien und -hyperplasien, eingesetzt werden. Darüber hinaus eignen sich die Substanzen zur diagnostischen Anwendung zur Erkennung von Krankheiten, die von einer gesteigerten Aktivität des Na⁺/H⁺-Antiporters z.B. in Erythrozyten, Thrombozyten oder Leukozyten begleitet werden.

Die Wirkungen der Verbindungen können mit Hilfe an sich bekannter Methoden ermittelt werden, wie sie z.B. von M. Escobales and J. Figueroa in J. Membrane Biol. 120, 41-49 (1991) oder von L. Counillon, W. Scholz, H.J. Lang und J. Pouysségur in Mol. Pharmacol. 44, 1041-1045 (1993) angegeben werden.

Als Versuchstiere eignen sich z.B. Mäuse, Ratten, Meerschweinchen, Hunde, Katzen, Affen oder Schweine.

Die Verbindungen können daher als Arzneimittelwirkstoffe in der Human-und Veterinärmedizin verwendet werden. Ferner können sie als Zwischenprodukte zur Herstellung weiterer Arzneimittelwirkstoffe Verwendung finden.

In den angegebenen Formeln bedeutet A eine verzweigte oder unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

R¹ bedeutet vorzugsweise A, OA oder Hal, insbesondere Br oder Cl. Ferner aber auch vorzugsweise CH₂F, CHF₂, CF₃ oder C₂F₅.

R² und R³ bedeuten vorzugsweise unabhängig voneinander H, A-SO₂, A, CF₃, Cl, Br, CN oder OA. Besonders bevorzugt steht einer der beiden Reste für H₃C-SO₂-, während der andere eine der zuvor genannten bevorzugten Bedeutungen besitzt oder aber vorzugsweise Wasserstoff ist. Einer der beiden Reste R² und R³ steht vorzugsweise in 3- oder 5-Position der Benzoylgruppe. Sofern einer der Reste A-SO₂- bedeutet, so befindet sich dieser vorzugsweise in der meta-Position zur Benzoylguanidin-Gruppe.

R⁴ bedeutet vorzugsweise, ebenso wie R⁵, H oder A.

Falls R⁴ und R⁵ zusammen Alkylen bedeuten, so ist die Alkylengruppe vorzugsweise unverzweigt, im einzelnen bevorzugt -(CH2)ₖ-, wobei k 4 oder 5 bedeutet; aber auch bevorzugt -(CH₂)₂-O-(CH₂)₂-, -(CH₂)₂-NH₋(CH₂)₂-, -(CH₂)₂-NA-(CH₂)₂-, -CH₂-O-(CH₂)₂-, -CH₂-NH-(CH₂)₂-, oder -CH₂-NA-(CH₂)₂- bzw. -CO-(CH₂)₃-, -CO-(CH₂)₄- oder -CH₂-CO-(CH₂)₂.

Ph bedeutet vorzugsweise unsubstituiertes oder einfach durch Cl, Br, A, OA, NH₂, NHA, NA₂ oder CF₃ substituiertes Phenyl.

R⁶ steht bevorzugt für A, insbesondere für Methyl oder aber vorzugsweise auch für unsubstituiertes Phenyl.

Der Rest X bedeutet vorzugsweise O oder S.

Alk bedeutet bevorzugt C₁ bis C₈-Alkyl oder C₃ bis C₈-Cycloalkyl. Sofern Alk nicht-cyclisch ist, steht der Rest vorzugsweise für einen der Alkylreste, die auch für A bevorzugt sind. Besonders bevorzugte Cycloalkylreste für die Alk stehen kann, sind Cyclopropyl, Cyclopentyl, Cyclohexyl oder deren einfach durch A, insbesondere Methyl, Ethyl oder Isopropyl, substituierte Abkömmlinge.

R⁷ und R^{7'} bedeuten vorzugsweise unabhängig voneinander H, A oder unsubstituiertes Phenyl.

Hal bedeutet vorzugsweise F, Cl oder Br.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-,2-, 3-, 4-, 5-, 6- oder 7-Indolyl, 1-,2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolinyl, 1-, 3-, 4-, 5-, 6-,7- oder 8-Isochinolinyl, 1-,2-, 3-, 4- oder 9-Carbazolyl, 1-,2-, 3-, 4-, 5-, 6-,7-, 8- oder 9-Acridinyl, 3-, 4-, 5-, 6-,7- oder 8-Cinnolinyl, 2-, 4-, 5-, 6- 7- oder 8-Chinazolinyl. Die heterocyclischen Reste können auch teilweise oder vollständig hydriert sein. Het kann also z.B. auch bedeuten 2,3-Dihydro-2-, -3-, 4- oder -5-furyl, 2,5-Dihydro-2-, - 3-, 4- oder -5-furyl, Tetrahydro-2- oder -3-furyl, 1,3-Dioxolan-4-yl, Tetrahydro-2- oder -3-thienyl, 2,3-Dihydro-1-, -2-, -3-, 4- oder -5-pyrrolyl, 2,5-Dihydro-1-, -2-, -3-, 4- oder -5-pyrrolyl, 1-,2- oder 3-Pyrrolidinyl, Tetrahydro-1-, -2- oder 4-imidazolyl, 2,3-Dihydro-1-, -2-, -3-, -4- oder -5-pyrazolyl, Tetrahydro-1-, -3- oder -4-pyrazolyl, 1 ,4-Dihydro-1-, -2-, -3-oder -4-pyridyl, 1,2,3,4-Tetrahydro-1-, -2-, -3-, -4-, -5- oder - 6-pyridyl, 1,2,3,6-Tetrahydro-1-, -2-, -3-, -4-, -5- oder -6-pyridyl, 1-,2-, 3- oder 4-Piperidinyl, 2-, 3- oder 4-Morpholinyl, Tetrahydro-2-, -3- oder 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-,2- oder 3-Piperazinyl, 1,2,3,4-Tetrahydro-1-, -2-, -3-, -4-, -5-, 6-, -7- oder -8-chinolinyl, 1,2,3,4-Tetrahydro-1-, -2-, -3-, -4-, -5-, -6-, -7- oder 8-isochinolinyl.

Allgemein gilt, daß sämtliche Reste wie z.B. Het oder Ph, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ih ausgedrückt werden, die der Formel I entsprechend und worin die nicht näher bezeichneten Reste die bei der Formel I angegebene Bedeutung haben, worin jedoch
- in Ia: R¹ A und R² -SO₂-A oder-SO₂-NH₂ bedeuten;
- in Ib: R¹ A oder Hal und Alk verzweigtes oder unverzweigtes Alkyl mit 1 bis 8 C-Atomen bedeutet;
- in Ic: R¹ A oder Hal und Alk Cycloalkyl mit 3 bis 8 C-Atomen bedeutet;
- in Id: Alk in para-Position zur Amidgruppe steht, R² -SO₂-A oder -SO₂NH₂ und R¹ A, OA oder Hal bedeutet;
- in Ie: Alk in para-Position zur Amidgruppe steht und Methyl, Ethyl, Propyl oder Isopropyl bedeutet und R² in meta-Position zur Amidgruppe steht und -SO₂-A bedeutet;
- in If: R¹ und R² benachbart zueinander sind und R¹ A, OA oder Hal und R² -SO₂-A bedeutet;
- in Ig: Alk in para-Position zur Amidgruppe steht und Cyclopropyl, Cyclopentyl oder Cyclohexyl und R² in meta-Position zur Amidgruppe steht und -SO₂-A bedeutet;
- in Ih: R¹, A, OA oder Hal, R² SO₂-A und Alk Methyl, Ethyl, Propyl, Isopropyl, Cyclopentyl oder Cyclohexyl bedeutet.

Gegenstand der Erfindung ist ferner ein Verfahren zur Herstellung der Verbindungen der Formel I gemäß Anspruch 1, sowie von deren Salzen, dadurch gekennzeichnet, daß man eine Verbindung der Formel II 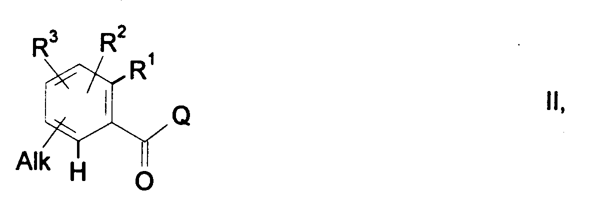 worin R¹, R², R³ und Alk die zuvor angegebenen Bedeutungen haben
und
- Q: Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt,
oder daß man ein Benzoylguanidin der Formel III 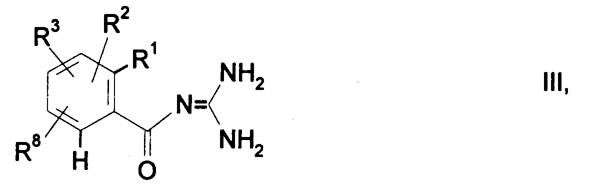 worin R¹, R² und R³ die zuvor angegebenen Bedeutungen besitzen,
und
- R⁸: F, Cl, Br, I oder H
bedeutet,
mit einer Verbindung der Formel IV

Alk-R^{8'} (IV)

worin
- Alk: die angegebene Bedeutung besitzt und
- R^{8'}: H, Cl, Br oder I bedeutet,
in Gegenwart eines Katalysators, nach vorheriger Metallierung bzw. Transmetallierung, umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man eine erhaltene Base der Formel I durch Behandeln mit einer Säure in eines ihrer Salze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in der oben angegebenen Patentanmeldung) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden Verbindungen der Formel I hergestellt, indem man ein aktiviertes Carbonsäurederivat der Formel II, wobei Q besonders bevorzugt Cl oder -O-CH₃ ist, mit Guanidin umsetzt. Besonders geeignet sind auch Reaktionsvarianten, bei denen die freie Carbonsäure II (Q = OH) in an sich bekannter Weise zu dem jeweiligen aktivierten Derivat umgesetzt und dieses dann direkt, ohne Zwischenisolierung, mit Guanidin zur Reaktion gebracht wird. Methoden, bei denen eine Zwischenisolierung entbehrlich ist, sind beispielsweise eine Aktivierung mit Carbonyldiimidazol, Dicyclohexylcarbodiimid oder die Mukayama-Variante (Angew. Chem. 91, 788-812 (1979).

Die Carbonsäuren und Carbonsäurederivate der Formel II sind in der Regel bekannt. Sie werden insbesondere durch Pd-katalysierte Kreuzkupplungen hergestellt. Bevorzugte Katalysatoren sind z.B. Pd(PPh₃)₄, (Ph₃P)₂PdCl₂, Pd(CH₃COO)₂ oder Pd-(II)-[1,1'-bis-diphenylphosphin)-ferrocen]-chlorid, vorzugsweise in Gegenwart von CuI.

Die Carbonsäuren der Formel II bzw. deren Derivate werden ferner hergestellt, indem man geeignete Benzoesäurederivate der Formel V 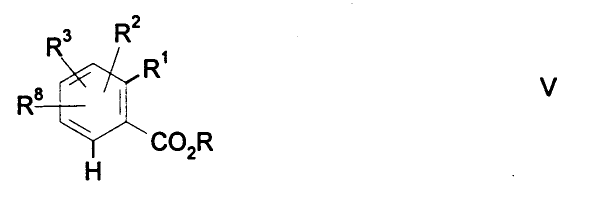 worin R¹, R², R³ und R⁸ die angegebenen Bedeutungen besitzen und R H oder A ist,
metalliert und dann mit eine Alkylhalogenid der Formel IV umsetzt. Eine geeignete Base zur Metallierung ist z.B. Lithiumdiisopropylamid.

Bei den zuvor genannten Kreuzkupplungen setzt man ein Carbonsäure- oder ein Esterderivat der Formel V, worin R⁸ Cl, Br oder I bedeutet, mit einer metallorganischen Alkylverbindung, die in situ durch Metallierung mit einem an sich bekannten Metallierungsreagenz aus einer Verbindung der Formel IV hergestellt wird, in Gegenwart eines geeigneten Metallkatalysators, insbesondere eines der zuvor genannten, um.

Die Umsetzung erfolgt in Analogie zu der Reaktion der Verbindungen III und IV. Sie wird nachstehend beschrieben.

Die Umsetzung eines reaktionsfähigen Carbonsäurederivates der Formel II mit Guanidin erfolgt in an sich bekannter Weise vorzugsweise in einem protischen oder aprotischen polaren oder unpolaren inerten organischen Lösungsmittel.

Geeignete Lösungsmittel werden nachfolgend für die Umsetzung der Verbindungen III und IV genannt. Besonders bevorzugte Solventien sind jedoch Methanol, THF, Dimethoxyethan, Dioxan oder daraus herstellbare Gemische sowie Wasser. Als Reaktionstemperatur sind beispielsweise Temperaturen zwischen 20° und dem Siedepunkt des Lösungsmittels geeignet. Die Reaktionszeiten liegen zwischen 5 Min. und 12 Std.. Es ist zweckmäßig, bei der Reaktion einen Säurefänger einzusetzen. Hierzu eignen sich jegliche Arten von Basen, die die Reaktion selbst nicht stören. Besonders geeignet ist jedoch die Verwendung von anorganischen Basen wie Kaliumcarbonat oder von organischen Basen wie Triethylamin oder Pyridin oder aber ein Überschuß des Guanidins.

Verbindungen der Formel I gemäß Anspruch 1 können ferner hergestellt werden, indem man ein Benzoylguanidin der Formel III mit einer Verbindung der Formel IV umsetzt. Die Ausgangsstoffe der Formel III können auf einfache Weise durch Umsetzung von entsprechend substituierten Benzoesäuren oder daraus ableitbaren reaktionsfähigen Säurederivaten, wie z.B. Säurehalogeniden, Estern oder Anhydriden, mit Guanidin, unter Reaktionsbedingungen wie sie für die Amidherstellung an sich bekannt und allgemein üblich sind, hergestellt werden. Besonders geeignet sind wiederum solche Reaktionsvarianten, wie sie zuvor für die Umsetzung von Verbindung II mit Guanidin angegeben werden.

Die Verbindungen der Formel IV sind ebenso wie die Methoden zu ihrer Herstellung an sich bekannt. Sofern sie nicht bekannt sind, können sie nach den an sich bekannten Methoden hergestellt werden.

Die Herstellung der Verbindung II sowie die Umsetzung der Verbindung III mit einer Verbindung der Formel IV erfolgt in an sich bekannter Weise, bevorzugt in einem protischen oder aprotischen polaren inerten organischen Lösungsmittel.

Bei der Herstellung von II oder bei der Umsetzung von III mit IV ist es ebenfalls zweckmäßig, in Gegenwart einer Base oder mit einem Überschuß der basischen Komponente zu arbeiten. Als Basen eignen sich bevorzugt z.B. Alkalimetall- oder Erdalkalimetallhydroxide, -carbonate, -alkoholate oder organische Basen wie Triethylamin oder Pyridin, die auch im Überschuß angewendet werden und dann gleichzeitig als Lösungsmittel dienen können.

Als inerte Lösungsmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Eine besonders bevorzugte Arbeitsweise bei der Umsetzung von III mit IV besteht darin, daß man das entsprechende Benzoylguanidin in einem inerten Lösungsmittel, wie z.B. Toluol, suspendiert mit einem Pd-(II)-Katalysator behandelt und anschließend tropfenweise die gewünschte zuvor transmetallierte Verbindung, z.B. eine Zn-Alkylverbindung, der Formel IV zusetzt.

Weiterhin können die Verbindungen der Formel I erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amine und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, insbesondere solche, die anstelle einer HN-Gruppe eine R'-N-Gruppe tragen, worin R' eine Aminoschutzgruppe bedeutet, und/oder solche, die anstelle des H-Atoms einer Hydroxygruppe eine Hydroxyschutzgruppe tragen, z.B. solche, die der Formel I entsprechen, jedoch anstelle einer OH-Gruppe eine OR''-Gruppe tragen, worin R'' eine Hydroxyschutzgruppe bedeutet.

Es können auch mehrere - gleiche oder verschiedene - geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden hergestellt werden, wie sie z.B. in den genannten Standardwerken und Patentanmeldungen beschrieben sind, z.B. durch Umsetzung von Verbindungen, die den Formeln II und III entsprechen, wobei jedoch mindestens eine dieser Verbindungen eine Schutzgruppe anstelle eines H-Atoms enthält.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran (THF) oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Eine Base der Formel I kann ferner mit einer Säure in das zugehörige Säureadditionssalz übergeführt werden. Für diese Umsetzung kommen Säuren in Frage, die physiologische unbedenkliche Salze liefern. So können anorganische Säuren verwendet werden, z.B. Schwefelsäure. Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure. Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure.

Die Verbindungen der Formel I und ihre physiologisch unbedenklichen Salze könne zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z.B. zur Herstellung von Injektionspräparaten verwendet werden.

Insbesondere für die topische Anwendung kommen auch liposomale Zubereitungen in Betracht. Die angegebenen Zubereitungen können sterilisiert sein und/oder Hilfsstoffe wie Geleit-, Konservierungs-, Stabilisierungs-und/oder Netzmittel, Emulgatoren, Salze zur Beeinflussung des osmotisches Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems. Sie eignen sich daher zur Behandlung von Arrhythmien, insbesondere wenn diese durch Sauerstoffmangel hervorgerufen werden, von Angina pectoris, Infarkten, Ischämien des Nervensystems wie z.B. Schlaganfall oder Hirnödeme, von Schockzuständen und zur Präventivbehandlung.

Die Substanzen können ferner als Therapeutika bei Erkrankungen eingesetzt werden, bei denen Zellproliferationen eine Rolle spielen wie Arteriosklerose, diabetische Spätkomplikationen, Tumorerkrankunken, Fibrosen sowie Organhypertrophien und -hyperplasien, insbesondere bei Erkrankungen der Prostata.

Dabei werden die erfindungsgemäßen Substanzen in der Regel in Analogie zu bekannten Antiarrhythmika, z.B. Aprindin, verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

### Beispiel 1

Man kocht eine Lösung von 1,4 g 2-Methyl-4-isopropyl-5-methylsulfonyl-benzoesäuremethylester [erhältlich durch Umsetzung von 2-Methyl-4-brom-5-methylsulfonyl-benzoesäuremethylester mit Isopropyl-zinkchlorid in Gegenwart von Pd-(II)-[1,1'-bis-(diphenylphosphin)-ferrocen]-chlorid und CuI] und 1,5 g Guanidin in 50 ml Methanol fünf Stunden und entfernt anschließend das Lösungsmittel. Der Rückstand wird mit Wasser behandelt, das verbleibende Kristallisat abgesaugt und mit verd. Natronlauge behandelt. Man filtriert den festen Rückstand ab, kristallisiert aus Ethanol um und erhält N-Diaminomethylen-2-methyl-4-isopropyl-5-methylsulfonyl-benzamid, F. 220-223°.

Analog erhält man durch Umsetzung von Guanidin
mit 2,4-Dimethyl-5-methylsulfonyl-benzoesäuremethylester das N-Diaminomethylen-2,4-dimethyl-5-methylsulfonyl-benzamid, F. 197°;
mit 2-Methyl-4-ethyl-5-methylsuffonyl-benzoesäuremethylester das N-Diaminomethylen-2-methyl-4-ethyl-5-methylsulfonyl-benzamid, F. 197-198°; F. 235-236° (Methansulfonat);
mit 2-Methyl-4-propyl-5-methylsulfonyl-benzoesäuremethylester das N-Diaminomethylen-2-methyl-4-propyl-5-methylsulfonyl-benzamid;
mit 2-Methyl-4-butyl-5-methylsulfonyl-benzoesäuremethylester das N-Diaminomethylen-2-methyl-4-butyl-5-methylsulfonyl-benzamid;
mit 2-Methyl-4-(2-butyl)-5-methylsulfonyl-benzoesäuremethylester das N-Diaminomethylen-2-methyl-4-(2-butyl)-5-methylsulfonyl-benzamid;
mit 2-Methyl-4-pentyl-5-methylsulfonyl-benzoesäuremethylester das N-Diaminomethylen-2-methyl-4-pentyl-5-methylsulfonyl-benzamid;
mit 2-Methyl-4-(2-pentyl)-5-methylsulfonyl-benzoesäuremethylester das N-Diaminomethylen-2-methyl-4-(2-pentyl)-5-methylsulfonyl-benzamid;
mit 2-Methyl-4-(3-pentyl)-5-methylsulfonyl-benzoesäuremethylester das N-Diaminomethylen-2-methyl-4-(3-pentyl)-5-methylsulfonyl-benzamid;
mit 2,3,4-Trimethyl-5-methylsulfonyl-benzoesäuremethylester das N-Diaminomethylen-2,3,4-trimethyl-5-methylsulfonylbenzamid;
mit 2-Methyl-4-hexyl-5-methylsulfonyl-benzoesäuremethylester das N-Diaminomethylen-2-methyl-4-hexyl-5-methylsulfonyl-benzamid;
mit 2-Methyl-4-(2-hexyl)-5-methylsulfonyl-benzoesäuremethylester das N-Diaminomethylen-2-methyl-4-(2-hexyl)-5-methylsulfonyl-benzamid;
mit 2-Methyl-4-cyclobutyl-5-methylsulfonyl-benzoesäuremethylester das N-Diaminomethylen-2-methyl-4-cyclobutyl-5-methylsulfonyl-benzamid;
mit 2-Methyl-4-cyclopropyl-5-methylsulfonyl-benzoesäuremethylester das N-Diaminomethylen-2-methyl-4-cyclopropyl-5-methylsulfonyl-benzamid;
mit 2-Methyl-4-cyclopentyl-5-methylsulfonyl-benzoesäuremethylester das N-Diaminomethylen-2-methyl-4-cyclopentyl-5-methylsulfonyl-benzamid, F. 120-125°; Methansulfonat F. 199-200°;
mit 2-Methyl-4-cyclohexyl-5-methylsulfonyl-benzoesäuremethylester das N-Diaminomethylen-2-methyl-4-cyclohexyl-5-methylsulfonyl-benzamid;
mit 2-Methyl-4-isobutyl-5-methylsulfonyl-benzoesäuremethylester das N-Diaminomethylen-2-methyl-4-isobutyl-5-methylsulfonyl-benzamid;
mit 2-Methyl-4-tert.-butyl-5-methylsulfonyl-benzoesäuremethylester das N-Diaminomethylen-2-methyl-4-tert.-butyl-5-methylsulfonyl-benzamid;
mit 2,4-Diethyl-5-methylsulfonyl-benzoesäuremethylester das N-Diaminomethylen-2,4-diethyl-5-methylsulfonyl-benzamid;
mit 2,3,4-Trimethyl-5-nitro-benzoesäuremethylester das N-Diaminomethylen-2,3,4-trimethyl-5-nitro-benzamid, F. 230°;
mit 2-Ethyl-4-methyl-5-methylsulfonyl-benzoesäuremethylester das N-Diaminomethylen-2-ethyl-4-methyl-5-methylsulfonyl-benzamid;
mit 2-Ethyl-4-isopropyl-5-methylsulfonyl-benzoesäuremethylester das N-Diaminomethylen-2-ethyl-4-isopropyl-5-methylsulfonyl-benzamid;
mit 2-Ethyl-4-propyl-5-methylsulfonyl-benzoesäuremethylester das N-Diaminomethylen-2-ethyl-4-propyl-5-methylsulfonyl-benzamid;
mit 2-Ethyl-4-butyl-5-methylsulfonyl-benzoesäuremethylester das N-Diaminomethylen-2-ethyl-4-butyl-5-methylsulfonyl-benzamid;
mit 2-Ethyl-4-(2-butyl)-5-methylsulfonyl-benzoesäuremethylester das N-Diaminomethylen-2-ethyl-4-(2-butyl)-5-methylsulfonyl-benzamid;
mit 2-Ethyl-4-pentyl-5-methylsulfonyl-benzoesäuremethylester das N-Diaminomethylen-2-ethyl-4-pentyl-5-methylsulfonyl-benzamid;
mit 2-Ethyl-4-(2-pentyl)-5-methylsulfonyl-benzoesäuremethylester das N-Diaminomethylen-2-ethyl-4-(2-pentyl)-5-methylsulfonyl-benzamid;
mit 2-Ethyl-4-(3-pentyl)-5-methylsulfonyl-benzoesäuremethylester das N-Diaminomethylen-2-ethyl-4-(3-pentyl)-5-methylsulfonyl-benzamid;
mit 2-Ethyl-4-hexyl-5-methylsulfonyl-benzoesäuremethylester das N-Diaminomethylen-2-ethyl-4-hexyl-5-methylsulfonyl-benzamid;
mit 2-Ethyl-4-(2-hexyl)-5-methylsulfonyl-benzoesäuremethylester das N-Diaminomethylen-2-ethyl-4-(2-hexyl)-5-methylsulfonyl-benzamid;
mit 2-Ethyl-4-(3-hexyl)-5-methylsulfonyl-benzoesäuremethylester das N-Diaminomethylen-2-ethyl-4-(3-hexyl)-5-methylsulfonyl-benzamid;
mit 2-Ethyl-4-cyclopropyl-5-methylsulfonyl-benzoesäuremethylester das N-Diaminomethylen-2-ethyl-4-cyclopropyl-5-methylsulfonyl-benzamid;
mit 2-Ethyl-4-cyclopenryl-5-methylsulfonyl-benzoesäuremethylester das N-Diaminomethylen-2-ethyl-4-cyclopentyl-5-methylsulfonyl-benzamid;
mit 2-Ethyl-4-cyclohexyl-5-methylsulfonyl-benzoesäuremethylester das N-Diaminomethylen-2-ethyl-4-cyclohexyl-5-methylsulfonyl-benzamid;
mit 2-Ethyl-4-(2-methylcyclopentyl)-5-methylsulfonyl-benzoesäuremethylester das N-Diaminomethylen-2-ethyl-4-(2-methylcyclopentyl)-5-methylsulfonyl-benzamid;
mit 2-Ethyl-4-(4-methylcyclohexyl)-5-methylsulfonyl-benzoesäuremethylester das N-Diaminomethylen-2-ethyl-4-(4-methylcyclohexyl)-5-methylsulfonyl-benzamid;
mit 2,4-Dipropyl-5-methylsulfonyl-benzoesäuremethylester das N-Diaminomethylen-2,4-dipropyl-5-methylsulfonyl-benzamid;
mit 2-Propyl-4-methyl-5-methylsulfonyl-benzoesäuremethylester das N-Diaminomethylen-2-propyl-4-methyl-5-methylsulfonyl-benzamid;
mit 2-Propyl-4-isopropyl-5-methylsulfonyl-benzoesäuremethylester das N-Diaminomethylen-2-propyl-4-isopropyl-5-methylsulfonyl-benzamid;
mit 2-Propyl-4-ethyl-5-methylsulfonyl-benzoesäuremethylester das N-Diaminomethylen-2-propyl-4-ethyl-5-methylsulfonyl-benzamid;
mit 2-Propyl-4-butyl-5-methylsulfonyl-benzoesäuremethylester das N-Diaminomethylen-2-propyl-4-butyl-5-methylsulfonyl-benzamid;
mit 2-Propyl-4-(2-butyl)-5-methylsulfonyl-benzoesäuremethylester das N-Diaminomethylen-2-propyl-4-(2-butyl)-5-methylsulfonyl-benzamid;
mit 2-Propyl-4-pentyl-5-methylsulfonyl-benzoesäuremethylester das N-Diaminomethylen-2-propyl-4-pentyl-5-methylsulfonyl-benzamid;
mit 2-Propyl-4-(2-pentyl)-5-methylsulfonyl-benzoesäuremethylester das N-Diaminomethylen-2-propyl-4-(2-pentyl)-5-methylsulfonyl-benzamid;
mit 2-Propyl-4-(3-pentyl)-5-methylsulfonyl-benzoesäuremethylester das N-Diaminomethylen-2-propyl-4-(3-pentyl)-5-methylsulfonyl-benzamid;
mit 2-Propyl-4-hexyl-5-methylsulfonyl-benzoesäuremethylester das N-Diaminomethylen-2-propyl-4-hexyl-5-methylsulfonyl-benzamid;
mit 2-Propyl-4-(2-hexyl)-5-methylsulfonyl-benzoesäuremethylester das N-Diaminomethylen-2-propyl-4-(2-hexyl)-5-methylsulfonyl-benzamid;
mit 2-Propyl-4-(3-hexyl)-5-methylsulfonyl-benzoesäuremethylester das N-Diaminomethylen-2-propyl-4-(3-hexyl)-5-methylsulfonylbenzamid;
mit 2-Propyl-4-cyclopropyl-5-methylsulfonyl-benzoesäuremethylester das N-Diaminomethylen-2-propyl-4-cyclopropyl-5-methylsulfonyl-benzamid;
mit 2-Propyl-4-cyclopentyl-5-methylsulfonyl-benzoesäuremethylester das N-Diaminomethylen-2-propyl-4-cyclopentyl-5-methylsulfonyl-benzamid;
mit 2-Propyl-4-cyclohexyl-5-methylsulfonyl-benzoesäuremethylester das N-Diaminomethylen-2-propyl-4-cyclohexyl-5-methylsulfonyl-benzamid;
mit 2-Propyl-4-(2-methylcyclopentyl)-5-methylsulfonyl-benzoesäuremethylester das N-Diaminomethylen-2-propyl-4-(2-methylcyclopentyl)-5-methylsulfonyl-benzamid;
mit 2-Propyl-4-(4-methylcyclohexyl)-5-methylsulfonyl-benzoesäuremethylester das N-Diaminomethylen-2-propyl-4-(4-methylcyclohexyl)-5-methylsulfonyl-benzamid.

### Beispiel 2

Analog Beispiel 1 erhält man durch Umsetzung von 1,8 g 2-Brom-4-methyl-5-methylsulfonyl-benzoesäuremethylester [erhältlich durch Umsetzung von 2,4-Dibrom-5-methylsulfonyl-benzoesäuremethyl-ester mit Methyl-zinkchlorid] mit 1,5 g Guanidin in Methanol das N-Diamino-methylen-2-brom-4-methyl-5-methylsulfonyl-benzamid.

Analog erhält man durch Umsetzung von Guanidin
mit 2-Fluormethyl-4-methyl-5-methylsulfonyl-benzoesäuremethylester das N-Diaminomethylen-2-fluormethyl-4-dimethyl-5-methylsulfonyl-benzamid;
mit 2-Difluormethyl-4-ethyl-5-methylsulfonyl-benzoesäuremethylester das N-Diaminomethylen-2-difluormethyl-4-ethyl-5-methylsulfonyl-benzamid;
mit 2-Brom-4-propyl-5-methylsulfonyl-benzoesäuremethylester das N-Diaminomethylen-2-brom-4-propyl-5-methylsulfonyl-benzamid;
mit 2-Chlor-4-butyl-5-methylsulfonyl-benzoesäuremethylester das N-Diaminomethylen-2-chlor-4-butyl-5-methylsulfonyl-benzamid;
mit 2-Brom-4-(2-butyl)-5-methylsulfonyl-benzoesäuremethylester das N-Diaminomethylen-2-brom-4-(2-butyl)-5-methylsulfonyl-benzamid;
mit 2-Trifluormethyl-4-pentyl-5-methylsulfonyl-benzoesäuremethylester das N-Diaminomethylen-2-trifluormethyl-4-pentyl-5-methylsulfonyl-benzamid;
mit 2-Fluormethyl-4-(2-pentyl)-5-methylsulfonyl-benzoesäuremethylester das N-Diaminomethylen-2-fluormethyl-4-(2-pentyl)-5-methylsulfonyl-benzamid;
mit 2-Brom-4-(3-pentyl)-5-methylsulfonyl-benzoesäuremethylester das N-Diaminomethylen-2-brom-4-(3-pentyl)-5-methylsulfonyl-benzamid;
mit 2-Chlor-4-hexyl-5-methylsulfonyl-benzoesäuremethylester das N-Diaminomethylen-2-chlor-4-hexyl-5-methylsulfonyl-benzamid;
mit 2-Difluormethyl-4-(2-hexyl)-5-methylsulfonyl-benzoesäuremethylester das N-Diaminomethylen-2-difluormethyl-4-(2-hexyl)-5-methylsulfonyl-benzamid;
mit 2-Brom-4-(3-hexyl)-5-methylsulfonyl-benzoesäuremethylester das N-Diaminomethylen-2-brom-4-(3-hexyl)-5-methylsulfonyl-benzamid;
mit 2-Brom-4-cyclopropyl-5-methylsulfonyl-benzoesäuremethylester das N-Diaminomethylen-2-brom-4-cyclopropyl-5-methylsulfonyl-benzamid;
mit 2-Brom-4-cyclopentyl-5-methylsulfonyl-benzoesäuremethylester das N-Diaminomethylen-2-brom-4-cyclopentyl-5-methylsulfonyl-benzamid;
mit 2-Brom-4-cyclohexyl-5-methylsulfonyl-benzoesäuremethylester das N-Diaminomethylen-2-brom-4-cyclohexyl-5-methylsulfonyl-benzamid;
mit 2-Brom-4-(2-methylcyclopentyl)-5-methylsulfonyl-benzoesäuremethylester das N-Diaminomethylen-2-brom-4-(2-methylcyclopentyl)-5-methylsulfonyl-benzamid;
mit 2-Brom-4-(4-methylcyclohexyl)-5-methylsulfonyl-benzoesäuremethylester das N-Diaminomethylen-2-brom-4-(4-methylcyclohexyl)-5-methylsulfonyl-benzamid;
mit 2-Cyan-4-ethyl-5-methylsulfonyl-benzoesäuremethylester das N-Diaminomethylen-2-cyan-4-ethyl-5-methylsulfonyl-benzamid;
mit 2-Cyan-4-methyl-5-methylsulfonyl-benzoesäuremethylester das N-Diaminomethylen-2-cyan-4-methyl-5-methylsulfonyl-benzamid;
mit 2-Cyan-4-isopropyl-5-methylsulfonyl-benzoesäuremethylester das N-Diaminomethylen-2-cyan-4-isopropyl-5-methylsulfonyl-benzamid;
mit 2-Cyan-4-propyl-5-methylsulfonyl-benzoesäuremethylester das N-Diaminomethylen-2-cyan-4-propyl-5-methylsulfonyl-benzamid;
mit 2-Nitro-4-butyl-5-methylsulfonyl-benzoesäuremethylester das N-Diaminomethylen-2-nitro-4-butyl-5-methylsulfonyl-benzamid;
mit 2-Ethinyl-4-(2-butyl)-5-methylsulfonyl-benzoesäuremethylester das N-Diaminomethylen-2-ethinyl-4-(2-butyl)-5-methylsulfonyl-benzamid;
mit 2-Ethinyl-4-pentyl-5-methylsulfonyl-benzoesäuremethylester das N-Diaminomethylen-2-ethinyl-4-pentyl-5-methylsulfonyl-benzamid;
mit 2-Cyan-4-(2-pentyl)-5-methylsulfonyl-benzoesäuremethylester das N-Diaminomethylen-2-cyan-4-(2-pentyl)-5-methylsulfonyl-benzamid;
mit 2-Nitro-4-(3-pentyl)-5-methylsulfonyl-benzoesäuremethylester das N-Diaminomethylen-2-nitro-4-(3-pentyl)-5-methylsulfonyl-benzamid;
mit 2-Ethinyl-4-hexyl-5-methylsulfonyl-benzoesäuremethylester das N-Diaminomethylen-2-ethinyl-4-hexyl-5-methylsulfonyl-benzamid;
mit 2-Cyan-4-(2-hexyl)-5-methylsulfonyl-benzoesäuremethylester das N-Diaminomethylen-2-cyan-4-(2-hexyl)-5-methylsulfonyl-benzamid;
mit 2-Pentafluorethyl-4-(3-hexyl)-5-methylsulfonyl-benzoesäuremethylester das N-Diaminomethylen-2-pentafluorethyl-4-(3-hexyl)-5-methylsulfonyl-benzamid;
mit 2-Cyan-4-cyclopropyl-5-methylsulfonyl-benzoesäuremethylester das N-Diaminomethylen-2-cyan-4-cyclopropyl-5-methylsulfonyl-benzamid;
mit 2-Ethinyl-4-cyclopentyl-5-methylsulfonyl-benzoesäuremethylester das N-Diaminomethylen-2-ethinyl-4-cyclopenryl-5-methylsulfonyl-benzamid;
mit 2-Cyan-4-cycloheryl-5-methylsulfonyl-benzoesäuremethylester das N-Diaminomethylen-2-cyan-4-cyclohexyl-5-methylsulfonyl-benzamid;
mit 2-Nitro-4-(2-methylcyclopentyl)-5-methylsulfonyl-benzoesäuremethylester das N-Diaminomethylen-2-nitro-4-(2-methylcyclopentyl)-5-methylsulfonyl-benzamid;
mit 2-Pentafluorethyl-4-(4-methylcyclohexyl)-5-methylsulfonyl-benzoesäuremethylester das N-Diaminomethylen-2-pentafluorethyl-4-(4-methylcyclohexyl)-5-methylsulfonyl-benzamid;
mit 2-Pentafluorethyl-4-propyl-5-methylsulfonyl-benzoesäuremethylester das N-Diaminomethylen-2-pentafluorethyl-4-propyl-5-methylsulfonyl-benzamid;
mit 2-Methoxy-4-methyl-5-methylsulfonyl-benzoesäuremethylester das N-Diaminomethylen-2-methoxy-4-methyl-5-methylsulfonyl-benzamid;
mit 2-Methoxy-4-isopropyl-5-methylsulfonyl-benzoesäuremethylester das N-Diaminomethylen-2-methoxy-4-isopropyl-5-methylsulfonyl-benzamid;
mit 2-Methoxy-4-ethyl-5-methylsulfonyl-benzoesäuremethylester das N-Diaminomethylen-2-methoxy-4-ethyl-5-methylsulfonyl-benzamid;
mit 2-Methoxy-4-butyl-5-methylsulfonyl-benzoesäuremetyylester das N-Diaminomethylen-2-methoxy-4-butyl-5-methylsulfonyl-benzamid;
mit 2-Methoxy-4-(2-butyl)-5-methylsulfonyl-benzoesäuremethylester das N-Diaminomethylen-2-methoxy-4-(2-butyl)-5-methylsulfonyl-benzamid;
mit 2-Methoxy-4-pentyl-5-methylsulfonyl-benzoesäuremethylester das N-Diaminomethylen-2-methoxy-4-pentyl-5-methylsulfonyl-benzamid;
mit 2-Methoxy-4-(2-pentyl)-5-methylsulfonyl-benzoesäuremethylester das N-Diaminomethylen-2-methoxy-4-(2-pentyl)-5-methylsulfonyl-benzamid;
mit 2-Methoxy-4-(3-pentyl)-5-methylsulfonyl-benzoesäuremethylester das N-Diaminomethylen-2-methoxy-4-(3-pentyl)-5-methylsulfonyl-benzamid;
mit 2-Methoxy-4-hexyl-5-methylsulfonyl-benzoesäuremethylester das N-Diaminomethylen-2-methoxy-4-hexyl-5-methylsulfonyl-benzamid;
mit 2-Methoxy-4-(2-hexyl)-5-methylsulfonyl-benzoesäuremethylester das N-Diaminomethylen-2-methoxy-4-(2-hexyl)-5-methylsulfonyl-benzamid;
mit 2-Methoxy-4-(3-hexyl)-5-methylsulfonyl-benzoesäuremethylester das N-Diaminomethylen-2-methoxy-4-(3-hexyl)-5-methylsulfonyl-benzamid;
mit 2-Methoxy-4-cyclopropyl-5-methylsulfonyl-benzoesäuremethylester das N-Diaminomethylen-2-methoxy-4-cyclopropyl-5-methylsulfonyl-benzamid;
mit 2-Methoxy-4-cyclopentyl-5-methylsulfonyl-benzoesäuremethylester das N-Diaminomethylen-2-methoxy-4-cyclopentyl-5-methylsulfonyl-benzamid;
mit 2-Methoxy-4-cycloheryl-5-methylsulfonyl-benzoesäuremethylester das N-Diaminomethylen-2-methoxy-4-cyclohexyl-5-methylsulfonyl-benzamid;
mit 2-Methoxy-4-(2-methylcyclopentyl)-5-methylsulfonyl-benzoesäuremethylester das N-Diaminomethylen-2-methoxy-4-(2-methylcyclopentyl)-5-methylsulfonyl-benzamid;
mit 2-Fluor-4-methyl-5-methylsulfonyl-benzoesäuremethylester das N-Diaminomethylen-2-fluor-4-methyl-5-methylsulfonyl-benzamid;
mit 2-Fluor-4-ethyl-5-methylsulfonyl-benzoesäuremethylester das N-Diaminoethylen-2-fluor-4-ethyl-5-methylsulfonyl-benzamid;
mit 2-Fluor-4-isopropyl-5-methylsulfonyl-benzoesäuremethylester das N-Diaminomethylen-2-fluor-4-isopropyl-5-methylsulfonyl-benzamid;
mit 2-Fluor-4-propyl-5-methylsulfonyl-benzoesäuremethylester das N-Diaminomethylen-2-fluor-4-propyl-5-methylsulfonyl-benzamid;
mit 2-Fluor-4-butyl-5-methylsulfonyl-benzoesäuremethylester das N-Diaminomethylen-2-fluor-4-butyl-5-methylsulfonyl-benzamid;
mit 2-Fluor-4-(2-butyl)-5-methylsulfonyl-benzoesäuremethylester das N-Diaminomethylen-2-fluor-4-(2-butyl)-5-methylsulfonyl-benzamid;
mit 2-Fluor-4-pentyl-5-methylsulfonyl-benzoesäuremethylester das N-Diaminomethylen-2-fluor-4-pentyl-5-methylsulfonyl-benzamid;
mit 2-Fluor-4-(2-pentyl)-5-methylsulfonyl-benzoesäuremethylester das N-Diaminomethylen-2-fluor-4-(2-pentyl)-5-methylsulfonyl-benzamid;
mit 2-Fluor-4-(3-pentyl)-5-methylsulfonyl-benzoesäuremethylester das N-Diaminomethylen-2-fluor-4-(3-pentyl)-5-methylsulfonyl-benzamid;
mit 2-Fluor-4-hexyl-5-methylsulfonyl-benzoesäuremethylester das N-Diaminometnyien-2-fluor-4-hexyl-5-methylsulfonyl-benzamid;
mit 2-Fluor-4-(2-hexyl)-5-methylsulfonyl-benzoesäuremethylester das N-Diaminomethylen-2-fluor-4-(2-hexyl)-5-methylsulfonyl-benzamid;
mit 2-Fluor-4-(3-hexyl)-5-methylsulfonyl-benzoesäuremethylester das N-Diaminomethylen-2-fluor-4-(3-hexyl)-5-methylsulfonyl-benzamid;
mit 2-Fluor-4-cyclopropyl-5-methylsulfonyl-benzoesäuremethylester das N-Diaminomethylen-2-fluor-4-cyclopropyl-5-methylsulfonyl-benzamid;
mit 2-Fluor-4-cyclopentyl-5-methylsulfonyl-benzoesäuremethylester das N-Diaminomethylen-2-fluor-4-cyclopentyl-5-methylsulfonyl-benzamid;
mit 2-Fluor-4-cyclohexyl-5-methylsulfonyl-benzoesäuremethylester das N-Diaminomethylen-2-fluor-4-cyclohexyl-5-methylsulfony-benzamid;
mit 2-Fluor-4-(2-methylcyclopentyl)-5-methylsulfonyl-benzoesäuremethylester das N-Diaminomethylen-2-fluor-4-(2-methylcyclopentyl)-5-methylsulfonyl-benzamid;
mit 2-Fluor-4-(4-methylcyclohexyl)-5-methylsulfonyl-benzoesäuremethylester das N-Diaminomethylen-2-fluor-4-(4-methylcyclohexyl)-5-methylsulfonyl-benzamid;
mit 2-Methoxy-4-(4-methylcyclohexyl)-5-methylsulfonyl-benzoesäuremethlylester das N-Diaminomethylen-2-methoxy-4-(4-methylcylohexyl)-5-methylsulfonyl-benzamid.

### Beispiel 3

Zu einer Suspension von 1 g N-Diaminomethylen-2,3-dimethyl-4-brom-5-methylsulfonyl-benzamid [erhältlich durch Umsetzung von 2,3-Dimethyl-4-brom-5-methylsulfonyl-benzoyichlorid mit Guanidin in Gegenwart von Triethylamin] in 100 ml THF fügt man nacheinander 100 mg Palladium(II) [1,1'-bis-(diphenylphosphin)-ferrocen]chlorid sowie 100 mg CuI hinzu. Anschließend tropft man 10 g Methyl-zinkchlorid [erhältlich aus Methyl-magnesiumchlorid durch Transmetallierung mit ZnCl₂-Etherat in THF], gelöst in 100 ml THF, hinzu und rührt 20 Std. bei Raumtemperatur. Nach Filtration, Entfernung des Lösungsmittels und üblicher Aufarbeitung erhält man das N-Diaminomethylen-2,3,4-trimethyl-5-methylsulfonyl-benzamid, woraus nach Behandlung mit verdünnter wäßriger HCl-Lösung oder Methansulfonsäure und Gefriertrocknung das entsprechende Hydrochlorid oder Methansulfonat erhalten wird.

### Beispiel 4

700 mg N-Diaminomethylen-2-brom-4-methyl-5-methylsulfonyl-benzamid [erhältlich nach Bsp. 2] werden in 50 ml Wasser suspendiert und unter Rühren mit 1,8 ml 1 n HCl versetzt. Nach Filtration und Lyophilisation erhält man erhält N-Diaminomethylen-2-brom-4-methyl-5-methylsulfonyl-benzamid; Hydrochlorid, F 232°.

Analog erhält man aus den freien Basen die folgenden Hydrochloride oder Methansulfonate:
N-Diaminomethylen-2-ethyl-4-methyl-5-methylsulfonyl-benzamid, Hydrochlorid;
N-Diaminomethylen-2-propyl-4-methyl-5-methylsulfonyl-benzamid, Hydrochlorid;
N-Diaminomethylen-2-isopropyl-4-ethyl-5-methylsulfony-benzamid, Hydrochlorid;
N-Diaminomethylen-2-butyl-4-methyl-5-methylsulfonyl-benzamid, Hydrochlorid;
N-Diaminomethylen-2-(2-butyl)-4-ethylphenyl)-5-methylsulfonyl-benzamid, Hydrochlorid;
N-Diaminomethylen-2-methyl-4-isopropyl-5-methylsulfonyl-benzamid, Methansulfonat, F. 191-194°;
N-Diaminomethylen-2-propyl-4-isopropyl-5-methylsulfonyl-benzamid, Hydrochlorid;
N-Diaminomethylen-2-isopropyl-4-methyl-5-methylsulfonyl-benzamid, Hydrochlorid;
N-Diaminomethylen-2-butyl-4-methyl-5-methylsulfonyl-benzamid, Hydrochlorid;
N-Diaminomethylen-2-ethyl-4-isopropyl-5-methylsulfonyl-benzamid, Hydrochlorid;
N-Diaminomethylen-2,4-diethyl-5-methylsulfonyl-benzamid, Hydrochlorid;
N-Diaminomethylen-2-chlor-4-methyl-5-methylsulfonyl-benzamid, Hydrochlorid;
N-Diaminomethylen-2-brom-4-ethyl-5-methylsulfonyl-benzamid, Hydrochlorid;
N-Diaminomethylen-2-fluormethyl-4-methyl-5-methylsulfonyl-benzamid, Hydrochlorid;
N-Diaminomethylen-2-trifluormethyl-4-ethyl-5-methylsulfonyl-benzamid, Hydrochlorid;
N-Diaminomethylen-2-pentafluorethyl-4-methyl-5-methylsulfonyl-benzamid, Hydrochlorid;
N-Diaminomethylen-2-methoxy-4-isopropyl-5-methylsulfonyl-benzamid, Hydrochlorid;
N-Diaminonmethylen-2-cyan-4-methyl-5-methylsulfonyl-benzamid, Hydrochlorid;
N-Diaminomethylen-2-nitro-4-methyl-5-methylsulfonyl-benzamid, Hydrochlorid;
N-Diaminomethylen-2-ethinyl-4-methyl-5-methylsulfonyl-benzamid, Hydrochlorid.

### Beispiel 5

Analog Beispiel 1 erhält man durch Umsetzung von 1,2 g 2,4-Dimethyl-3-methylsulfonyl-benzoesäuremethylester [erhältlich durch Umsetzung von 2-Methyl-3-methylsulfonyl-4-brom-benzoesäuremethylester mit Methyl-zinkchlorid] mit 1,5g Guanidin in Methanol das N-Diaminomethylen-2,4-dimethyl-3-methylsulfonyl-benzamid.

Analog erhält man durch Umsetzung von Guanidin
mit 2-Methyl-3-methylsulfonyl-4-isopropyl-benzoesäuremethylester das N-Diaminomethylen-2-methyl-3-methylsulfonyl-4-isopropyl-benzamid;
mit 2-Methyl-3-methylsulfonyl-4-ethyl-benzoesäuremethylester das N-Diaminomethylen-2-methyl-3-methylsulfonyl-4-ethyl-benzamid;
mit 2-Methyl-3-methylsulfonyl-4-propyl-benzoesäuremethlester das N-Diaminomethylen-2-methyl-3-methylsulfonyl-4-propyl-benzamid;
mit 2-Methyl-3-methylsulfonyl-4-butyl-benzoesäuremethylester das N-Diaminomethylen-2-methyl-3-methylsulfonyl-4-butyl-benzamid;
mit 2-Methyl-3-methylsuffonyl-4-(2-butyl)-benzoesäuremethylester das N-Diaminomethylen-2-methyl-3-methylsulfonyl-4-(2-butyl)-benzamid;
mit 2-Methyl-3-methylsulfonyl-4-pentyl-benzoesäuremethylester das N-Diaminomethylen-2-methyl-3-methylsulfonyl-4-pentyl-benzamid;
mit 2-Methyl-3-methylsulfonyl-4-(2-pentyl)-benzoesäuremethylester das N-Diaminomethylen-2-methyl-3-methylsulfonyl-4-(2-pentyl)-benzamid;
mit 2-Methyl-3-methylsulfonyl-4-(3-pentyl)-benzoesäuremethylester das N-Diaminomethylen-2-methyl-3-methylsulfonyl-4-(3-pentyl)-benzamid;
mit 2-Methyl-3-methylsulfonyl-4-hexyl-benzoesäuremethylester das N-Diaminomethylen-2-methyl-3-methylsulfonyl-4-hexyl-benzamid;
mit 2-Methyl-3-methylsulfonyl-4-(2-hexyl)-benzoesäuremethylester das N-Diaminomethylen-2-methyl-3-methylsulfonyl-4-(2-hexyl)-benzamid;
mit 2-Methyl-3-methylsulfonyl-4-(3-hexyl)-benzoesäuremethylester das N-Diaminomethylen-2-methyl-3-methylsulfonyl-4-(3-hexyl)-benzamid;
mit 2-Methyl-3-methylsulfonyl-4-cyclopropyl-benzoesäuremethylester das N-Diaminomethylen-2-methyl-3-methylsulfonyl-4-cyclopropyl-benzamid;
mit 2-Methyl-3-methylsulfonyl-4-cyclopentyl-benzoesäuremethylester das N-Diaminomethylen-2-methyl-3-methylsulfonyl-4-cyclopentyl-benzamid;
mit 2-Methyl-3-methylsulfonyl-4-cyclohexyl-benzoesäuremethylester das N-Diaminomethylen-2-methyl-3-methylsulfonyl-4-cyclohexyl-benzamid;
mit 2-Methyl-3-methylsulfonyl-4-(2-methylcyclopentyl)-benzoesäuremethylester das N-Diaminomethylen-2-methyl-3-methylsulfonyl-4-(2-methylcyclopentyl)-benzamid;
mit 2-Methyl-3-methylsulfonyl-4-(4-methylcyclohexyl)-benzoesäuremethylester das N-Diaminomethylen-2-methyl-3-methylsulfonyl-4-(4-methylcyclohexyl)-benzamid;
mit 2-Ethyl-3-methylsulfonyl-4-methyl-benzoesäuremethylester das N-Diaminomethylen-2-ethyl-3-methylsulfonyl-4-methyl-benzamid;
mit 2-Ethyl-3-methylsulfonyl-4-isopropyl-benzoesäuremethylester das N-Diaminomethylen-2-ethyl-3-methylsulfonyl-4-isopropyl-benzamid;
mit 2-Ethyl-3-methylsulfonyl-4-propyl-benzoesäuremethylester das N-Diaminomethylen-2-ethyl-3-methylsulfonyl-4-propyl-benzamid;
mit 2-Ethyl-3-methylsulfonyl-4-butyl-benzoesäuremethylester das N-Diaminomethylen-2-ethyl-3-methylsulfonyl-4-butyl-benzamid;
mit 2-Ethyl-3-methylsulfonyl-4-(2-butyl)-benzoesäuremethylester das N-Diaminomethylen-2-ethyl-3-methylsulfonyl-4-(2-butyl)-benzamid;
mit 2-Ethyl-3-methylsulfonyl-4-pentyl-benzoesäuremethylester das N-Diaminomethylen-2-ethyl-3-methylsulfonyl-4-pentyl-benzamid;
mit 2-Ethyl-3-methylsulfonyl-4-(2-pentyl-benzoesäuremethylester das N-Diaminomethylen-2-ethyl-3-methylsulfonyl-4-(2-pentyl)-benzamid;
mit 2-Ethyl-3-methylsulfonyl-4-(3-pentyl-benzoesäuremethylester das N-Diaminomethylen-2-ethyl-3-methylsulfonyl-4-(3-pentyl)-benzamid;
mit 2-Ethyl-3-methylsulfonyl-4-hexyl-benzoesäuremethylester das N-Diaminomethylen-2-ethyl-3-methylsulfonyl-4-hexyl-benzamid;
mit 2-Ethyl-3-methylsulfonyl-4-(2-hexyl)-benzoesäuremethylester das N-Diaminomethylen-2-ethyl-3-methylsulfonyl-4-(2-hexyl)-benzamid;
mit 2-Ethyl-3-methylsulfonyl-4-(3-hexyl)-benzoesäuremethylester das N-Diaminomethylen-2-ethyl-3-methylsulfonyl-4-(3-hexyl)-benzamid;
mit 2-Ethyl-3-methylsulfonyl-4-cyclopropyl-benzoesäuremethylester das N-Diaminomethylen-2-ethyl-3-methylsulfonyl-4-cyclopropyl-benzamid;
mit 2-Ethyl-3-methylsulfonyl-4-cyclopentyl-benzoesäuremethylester das N-Diaminomethylen-2-ethyl-3-methylsulfonyl-4-cyclopentyl-benzamid;
mit 2-Ethyl-3-methylsulfonyl-4-cyclohexyl-benzoesäuremethylester das N-Diaminomethylen-2-ethyl-3-methylsulfonyl-4-cyclohexyl-benzamid;
mit 2-Ethyl-3-methylsulfonyl-4-(2-methylcyclopentyl)-benzoesäuremethylester das N-Diaminomethylen-2-ethyl-3-methylsulfonyl-4-(2-methylcyclopentyl)-benzamid;
mit 2-Ethyl-3-methylsulfonyl-4-(4-methylcyclohexyl)-benzoesäuremethylester das N-Diaminomethylen-2-ethyl-3-methylsulfonyl-4-(4-methylcyclohexyl)-benzamid;
mit 2-Propyl-3-methylsulfonyl-4-methyl-benzoesäuremethylester das N-Diaminomethylen-2-propyl-3-methylsulfonyl-4-methyl-benzamid;
mit 2-Propyl-3-methylsulfonyl-4-isopropyl-benzoesäuremethylester das N-Diaminomethylen-2-propyl-3-methylsulfonyl-4-isopropyl-benzamid;
mit 2-Propyl-3-methylsulfonyl-4-ethyl-benzoesäuremethylester das N-Diaminomethylen-2-propyl-3-methylsulfonyl-4-ethyl-benzamid;
mit 2-Propyl-3-methylsulfonyl-4-butyl-benzoesäuremethylester das N-Diaminomethylen-2-propyl-3-methylsulfonyl-4-butyl-benzamid;
mit 2-Propyl-3-methylsulfonyl-4-(2-butyl)-benzoesäuremethylester das N-Diaminomethylen-2-propyl-3-methylsulfonyl-4-(2-butyl)-benzamid;
mit 2-Propyl-3-methylsulfonyl-4-pentyl-benzoesäuremethylester das N-Diaminomethylen-2-propyl-3-methylsulfonyl-4-pentyl-benzamid;
mit 2-Propyl-3-methylsulfonyl-4-(2-pentyl)-benzoesäuremethylester das N-Diaminomethylen-2-propyl-3-methylsulfonyl-4-(2-pentyl)-benzamid;
mit 2-Propyl-3-methylsulfonyl-4-(3-pentyl)-benzoesäuremethylester das N-Diaminomethylen-2-propyl-3-methylsulfonyl-4-(3-pentyl)-benzamid;
mit 2-Propyl-3-methylsulfonyl-4-hexyl-benzoesäuremethylester das N-Diaminomethylen-2-propyl-3-methylsulfonyl-4-hexyl-benzamid;
mit 2-Propyl-3-methylsulfonyl-4-(2-hexyl)-benzoesäuremethylester das N-Diaminomethylen-2-propyl-3-methylsulfonyl-4-(2-hexyl)-benzamid;
mit 2-Propyl-3-methylsulfonyl-4-(3-hexyl)-benzoesäuremethylester das N-Diaminomethylen-2-propyl-3-methylsulfonyl-4-(3-hexyl)-benzamid;
mit 2-Propyl-3-methylsulfonyl-4-cyclopropyl-benzoesäuremethylester das N-Diaminomethylen-2-propyl-3-methylsulfonyl-4-cyclopropyl-benzamid;
mit 2-Propyl-3-methylsulfonyl-4-cyclopentyl-benzoesäuremethylester das N-Diaminomethylen-2-propyl-3-methylsulfonyl-4-cyclopentyl-benzamid;
mit 2-Propyl-3-methylsulfonyl-4-cyclohexyl-benzoesäuremethylester das N-Diaminomethylen-2-propyl-3-methylsulfonyl-4-cyclohexyl-benzamid;
mit 2-Propyl-3-methylsulfonyl-4-(2-methylcyclopentyl)-benzoesäuremethylester das N-Diaminomethylen-2-propyl-3-methylsulfonyl-4-(2-methylcyclopentyl)-benzamid;
mit 2-Propyl-3-methylsulfonyl-4-(4-methylcyclohexyl)-benzoesäuremethylester das N-Diaminomethylen-2-propyl-3-methylsulfonyl-4-(4-methylcyclohexyl)-benzamid;
mit 2-Fluormethyl-3-methylsulfonyl-4-methyl-benzoesäuremethylester das N-Diaminomethylen-2-fluormethyl-3-methylsulfonyl-4-dimethyl-benzamid;
mit 2-Difluormethyl-3-methylsulfonyl-4-ethyl-benzoesäuremethylester das N-Diaminomethylen-2-difluormethyl-3-methylsulfonyl-4-ethyl-benzamid;
mit 2-Brom-3-methylsulfonyl-4-propyl-benzoesäuremethylester das N-Diaminomethylen-2-brom-3-methylsulfonyl-4-propyl-benzamid;
mit 2-Chlor-3-methylsulfonyl-4-butyl-benzoesäuremethylester das N-Diaminomethylen-2-chlor-3-methylsulfonyl-4-butyl-benzamid;
mit 2-Brom-3-methylsulfonyl-4-(2-butyl)-benzoesäuremethylester das N-Diaminomethylen-2-brom-3-methylsulfonyl-4-(2-butyl)-benzamid;
mit 2-Trifluormethyl-3-methylsulfonyl-4-pentyl-benzoesäuremethylester das N-Diaminomethylen-2-trifluormethyl-3-methylsulfonyl-4-pentyl-benzamid;
mit 2-Fluormethyl-3-methylsulfonyl-4-(2-pentyl)-benzoesäuremethylester das N-Diaminomethylen-2-fluormethyl-3-methylsulfonyl-4-(2-pentyl)-benzamid;
mit 2-Brom-3-methylsulfonyl-4-(3-pentyl)-benzoesäuremethylester das N-Diaminomethylen-2-brom-3-methylsulfonyl-4-(3-pentyl)-benzamid;
mit 2-Chlor-3-methylsulfonyl-4-hexyl-benzoesäuremethylester das N-Diaminomethylen-2-chlor-3-methylsulfonyl-4-hexyl-benzamid;
mit 2-Difluormethyl-3-methylsulfonyl-4-(2-hexyl)-benzoesäuremethylester das N-Diaminomethylen-2-difluormethyl-3-methylsulfonyl-4-(2-hexyl)-benzamid;
mit 2-Brom-3-methylsulfonyl-4-(3-hexyl)-benzoesäuremethylester das N-Diaminomethylen-2-brom-3-methylsulfonyl-4-(3-hexyl)-benzamid;
mit 2-Brom-3-methylsulfonyl-4-cyclopropyl-benzoesäuremethylester das N-Diaminomethylen-2-brom-3-methylsulfonyl-4-cyclopropyl-benzamid;
mit 2-Brom-3-methylsulfonyl-4-cyclopentyl-benzoesäuremethylester das N-Diaminomethylen-2-brom-3-methylsulfonyl-4-cyclopentyl-benzamid;
mit 2-Brom-3-methylsulfonyl-4-cyclohexyl-benzoesäuremethylester das N-Diaminomethylen-2-brom-3-methylsulfonyl-4-cyclohexyl-benzamid;
mit 2-Brom-3-methylsulfonyl-4-(2-methylcyclopentyl)-benzoesäuremethylester das N-Diaminomethylen-2-brom-3-methylsulfonyl-4-(2-methylcyclopentyl)-benzamid;
mit 2-Brom-3-methylsulfonyl-4-(4-methylcyclohexyl)-benzoesäuremethylester das N-Diaminomethylen-2-brom-3-methylsulfonyl-4-(4-methylcyclohexyl)-benzamid;
mit 2-Cyan-3-methylsulfonyl-4-ethyl-benzoesäuremethylester das N-Diaminomethylen-2-cyan-3-methylsulfonyl-4-ethyl-benzamid;
mit 2-Cyan-3-methylsulfonyl-4-methyl-benzoesäuremethylester das N-Diaminomethylen-2-cyan-3-methylsulfonyl-4-methyl-benzamid;
mit 2-Cyan-3-methylsulfonyl-4-isopropyl-benzoesäuremethylester das N-Diaminomethylen-2-cyan-3-methylsulfonyl-4-isopropyl-benzamid;
mit 2-Cyan-3-methylsulfonyl-4-propyl-benzoesäuremethylester das N-Diaminomethylen-2-cyan-3-methylsulfonyl-4-propyl-benzamid;
mit 2-Nitro-3-methylsulfonyl-4-butyl-benzoesäuremethylester das N-Diaminomethylen-2-nitro-3-methylsulfonyl-4-butyl-benzamid;
mit 2-Ethinyl-3-methylsulfonyl-4-(2-butyl)-benzoesäuremethylester das N-Diaminomethylen-2-ethinyl-3-methylsulfonyl-4-(2-butyl)-benzamid;
mit 2-Ethinyl-3-methylsulfonyl-4-pentyl-benzoesäuremethylester das N-Diaminomethylen-2-ethinyl-3-methylsulfonyl-4-pentyl-benzamid;
mit 2-Cyan-3-methylsulfonyl-4-(2-pentyl)-benzoesäuremethylester das N-Diaminomethylen-2-cyan-3-methylsulfonyl-4-(2-pentyl)-benzamid;
mit 2-Fluor-3-methylsulfonyl-4-methyl-benzoesäuremethylester das N-Diaminomethylen-2-fluor-3-methylsulfonyl-4-methyl-benzamid;
mit 2-Fluor-3-methylsulfonyl-4-ethyl-benzoesäuremethylester das N-Diaminomethylen-2-fluor-3-methylsulfonyl-4-ethyl-benzamid;
mit 2-Fluor-3-methylsulfonyl-4-isopropyl-benzoesäuremethylester das N-Diaminomethylen-2-fluor-3-methylsulfon-4-isopropyl-benzamid;
mit 2-Fluor-3-methylsulfonyl-4-prop-benzoesäuremethylester das N-Diaminomethylen-2-fluor-3-methylsulfonyl-4-propyl-benzamid;
mit 2-Fluor-3-methylsulfonyl-4-butyl-benzoesäuremethylester das N-Diaminomethylen-2-fluor-3-methylsulfonyl-4-butyl-benzamid;
mit 2-Fluor-3-methylsulfonyl-4-(2-butyl)-benzoesäuremethylester das N-Diaminomethylen-2-fluor-3-methylsulfonyl-4-(2-butyl)-benzamid;
mit 2-Fluor-3-methylsulfonyl-4-pentyl-benzoesäuremethylester das N-Diaminomethylen-2-fluor-3-methylsulfonyl-4-pentyl-benzamid;
mit 2-Fluor-3-methylsulfonyl-4-(2-pentyl)-benzoesäuremethylester das N-Diaminomethylen-2-fluor-3-methylsulfonyl-4-(2-pentyl)-benzamid;
mit 2-Fluor-3-methylsulfonyl-4-(3-pentyl)-benzoesäuremethylester das N-Diaminomethylen-2-fluor-3-methylulfonyl-4-(3-pentyl)-benzamid;
mit 2-Fluor-3-methylsulfonyl-4-hexyl-benzoesäuremethylester das N-Diaminomethylen-2-fluor-3-methylsulfonyl-4-hexyl-benzamid;
mit 2-Fluor-3-methylsulfonyl-4-(2-hexyl)-benzoesäuremethylester das N-Diaminomethylen-2-fluo-3-methylsulfonyl-4-(2-hexyl)-benzamid;
mit 2-Fluo-3-methylsulfonyl-4-(3-hexyl)-benzoesäuremethylester das N-Diaminomethylen-2-fluor-3-methylsulfonyl-4-(3-hexyl)-benzamid;
mit 2-Fluor-3-methylsulfonyl-4-cyclopropyl-benzoesäuremethylester das N-Diaminomethylen-2-fluor-methylsulfony-4-cyclpropyl-benzamid;
mit 2-Fluor-3-methylsulfonyl-4-cyclopentyl-benzoesäuremethylester das N-Diaminomethylen-2-fluor-3-methylsulfonyl-4-cyclopentyl-benzamid;
mit 2-Fluor-3-methylsulfonyl-4-cyclohexyl-benzoesäuremethylester das N-Diaminomethylen-2-fluor-3-methylsulfonyl-4-cyclohexyl-benzamid;
mit 2-Fluor-3-methylsulfonyl-4-(2-methylcyclopentyl)-benzoesäuremethylester das N-Diaminomethylen-2-fluor-3-methylsulfonyl-4-(2-methylcyclopentyl)-benzamid;
mit 2-Fluor-3-methylsulfonyl-4-(4-methylcyclohexyl)-benzoesäuremethylester das N-Diaminomethylen-2-fluor-3-methylsulfonyl-4-(4-methylcyclohexyl)-benzamid;
mit 2-Nitro-3-methylsulfonyl-4-(3-pentyl)-benzoesäuremethylester das N-Diaminomethylen-2-nitro-3-methylsulfonyl-4-(3-pentyl)-benzamid;
mit 2-Ethinyl-3-methylsulfonyl-4-hexyl-benzoesäuremethylester das N-Diaminomethylen-2-ethinyl-3-methylsulfonyl-4-hexyl-benzamid;
mit 2-Cyan-3-methylsulfonyl-4-(2-hexyl)-benzoesäuremethylester das N-Diaminomethylen-2-cyan-3-methylsulfonyl-4-(2-hexyl)-benzamid;
mit 2-Pentafluorethyl-3-methylsulfonyl-4-(3-hexyl)-benzoesäuremethylester das N-Diaminomethylen-2-pentafluorethyl-3-methylsulfonyl-4-(3-hexyl)-benzamid;
mit 2-Cyan-3-methylsulfonyl-4-cyclopropyl-bezoesäuremethylester das N-Diaminomethylen-2-cyan-3-methylsulfonyl-4-cyclopropyl-benzamid;
mit 2-Ethinyl-3-methylsulfonyl-4-cyclopentyl-benzoesäuremethylester das N-Diaminomethylen-2-ethinyl-3-methylsulfonyl-4-cyclopentyl-benzamid;
mit 2-Cyan-3-methylsulfonyl-4-cyclohexyl-benzoesäuremethylester das N-Diaminomethylen-2-cyan-3-methylsulfonyl-4-cyclohexyl-benzamid;
mit 2-Nitro-3-methylsulfonyl-4-(2-methylcyclopentyl)-benzoesäuremethylester das N-Diaminomethylen-2-nitro-3-methylsulfonyl-4-(2-methylcyclopentyl)-benzamid;
mit 2-Pentafluorethyl-3-methylsulfonyl-4-(4-methylcyclohexyl)-benzoesäuremethylester das N-Diaminomethylen-2-pentafluorethyl-3-methylsulfonyl-4-(4-methylcyclohexyl)-benzamid;
mit 2-Pentafluorethyl-3-methylsulfonyl-4-propyl-benzoesäuremethylester das N-Diaminomethylen-2-pentafluorethyl-3-methylsulfonyl-4-propyl-benzamid;
mit 2-Methoxy-3-methylsulfonyl-4-methyl-benzoesäuremethylester das N-Diaminomethylen-2-methoxy-3-methylsulfonyl-4-methyl-benzamid;
mit 2-Methoxy-3-methylsulfonyl-4-isopropyl-benzoesäuremethylester das N-Diaminomethylen-2-methoxy-3-methylsulfonyl-4-isoproyl-benzamid;
mit 2-Methoxy-3-methylsulfonyl-4-ethyl-benzoesäuremethylester das N-Diaminomethylen-2-methoxy-3-methylsulfonyl-4-ethyl-benzamid;
mit 2-Methoxy-3-methylsulfonyl-4-butyl-benzoesäuremethylester das N-Diaminomethylen-2-methoxy-3-methylsulfonyl-4-butyl-benzamid;
mit 2-Methoxy-3-methylsulfonyl-4-(2-butyl)-benzoesäuremethylester das N-Diaminomethylen-2-methoxy-3-methylsulfonyl-4-(2-butyl)-benzamid;
mit 2-Methoxy-3-methylsulfonyl-4-pentyl-benzoesäuremethylester das N-Diaminomethylen-2-methoxy-3-methylsulfonyl-4-pentyl-benzamid;
mit 2-Methoxy-3-methylsulfonyl-4-(2-pentyl)-benzoesäuremethylester das N-Diaminomethylen-2-methoxy-3-methylsulfonyl-4-(2-pentyl)-benzamid;
mit 2-Methoxy-3-methylsulfonyl-4-(3-pentyl)-benzoesäuremethylester das N-Diaminomethylen-2-methoxy-3-methylsulfonyl-4-(3-pentyl)-benzamid;
mit 2-Methoxy-3-methylsulfonyl-4-hexyl-benzoesäuremethylester das N-Diaminomethylen-2-methoxy-3-methylsulfonyl-4-hexyl-benzamid;
mit 2-Methoxy-3-methylsulfonyl-4-(2-hexyl)-benzoesäuremethylester das N-Diaminomethylen-2-methoxy-3-methylsulfonyl-4-(2-hexyl)-benzamid;
mit 2-Methoxy-3-methylsulfonyl-4-(3-hexyl)-benzoesäuremethylester das N-Diaminomethylen-2-methoxy-3-methylsulfonyl-4-(3-hexyl)-benzamid;
mit 2-Methoxy-3-methylsulfonyl-4-cyclopropyl-benzoesäuremethylester das N-Diaminomethylen-2-methoxy-3-methylsulfonyl-4-cyclopropyl-benzamid;
mit 2-Methoxy-3-methylsulfonyl-4-cyclopentyl-benzoesäuremethylester das N-Diaminomethylen-2-methoxy-3-methylsulfonyl-4-cyclopentyl-benzamid;
mit 2-Methoxy-3-methylsulfonyl-4-cyclohexyl-benzoesäuremethylester das N-Diaminomethylen-2-methoxy-3-methylsulfonyl-4-cyclohexyl-benzamid;
mit 2-Methoxy-3-methylsulfonyl-4-(2-methylcyclopentyl)-benzoesäuremethylester das N-Diaminomethylen-2-methoxy-3-methylsulfonyl-4-(2-methylcyclopentyl)-benzamid;
mit 2-Methoxy-3-methylsulfonyl-4-(4-methylcylohexyl)-benzoesäuremethylester das N-Diaminomethylen-2-methoxy-3-methylsulfonyl-4-(4-methylcyclohexyl)-benzamid.

### Beispiel 6

Zu einer Suspension von 1 g N-Diaminomethylen-2-methoxy-3-methyl-4-brom-5-methylsulfonyl-benzamid [erhältlich durch Umsetzung von 2-Methoxy-3-methyl-4-brom-5-methylsulfonyl-benzoyl-chlorid mit Guanidin in Gegenwart von Triethylamin] in 70 ml THF fügt man nacheinander 100 mg Pd-(II)-[1,1'-bis-(diphenyl-phosphin)-ferrocen]-chlorid, 100 mg CuI sowie 10 Equivalente Cyclopentyl-zinkchlorid, gelöst in 10 ml THF hinzu und rührt 20 Stunden bei Raumtemperatur. Anschließend erhält man nach Filtration, Entfernung des Lösungsmittels und üblicher Aufarbeitung das N-Diaminomethy-len-2-methoxy-3-methyl-4-cyclopentyl-5-methylsulfonyl-benzamid, woraus nach Behandlung mit verdünnter wäßriger HCl-Lösung und Gefriertrocknung das entsprechende Hydrochlorid erhalten wird.

### Beispiel 7

Analog Beispiel 1 erhält man durch Umsetzung von 1,0 g 2-Ethyl-3-aminosulfonyl-4-methyl-benzoesäuremethylester [erhältlich durch Umsetzung von 2-Ethyl-3-aminosulfonyl-4-brom-benzoesäuremethylester mit Methylzinkchlorid in Gegenwart von Bis-(Triphenylphosphin)-palladium(II)-chlorid mit 0,9 g Guanidiniumchlorid das N-Diaminomethylen-2-ethyl-3-aminosulfonyl-4-methyl-benzamid.

Analog erhält man
aus 2-Methyl-3-aminosulfonyl-4-isopropyl-benzoesäuremethylester das N-Diaminomethylen-2-methyl-3-aminosulfonyl-4-isopropyl-benzamid;
aus 2-Ethyl-3-aminosulfonyl-4-methyl-benzoesäuremethylester das N-Diaminomethylen-2-ethyl-3-aminosulfonyl-4-methyl-benzamid;
aus 2-Cyan-3-aminosulfonyl-4-methyl-benzoesäuremethylester das N-Diaminomethylen-2-cyan-3-aminosulfonyl-4-methyl-benzamid;
aus 2-Ethyl-3-aminosulfonyl-4-propyl-benzoesäuremethylester das N-Diaminomethylen-2-ethyl-3-aminosulfonyl-4-propyl-benzamid;
aus 2-Brom-3-aminosulfonyl-4-ethyl-benzoesäuremethylester das N-Diaminomethylen-2-brom-3-aminosulfonyl-4-ethyl-benzamid;
aus 2-Difluormethyl-3-aminosulfonyl-4-methyl-benzoesäuremethylester das N-Diaminomethylen-2-difluormethyl-3-aminosulfonyl-4-ethyl-benzamid;
aus 2-Fluormethyl-3-aminosulfonyl-4-methyl-benzoesäuremethylester das N-Diaminomethylen-2-fluormethyl-3-aminosulfonyl-4-methyl-benzamid.

### Beispiel 8

Analog Beispiel 1 erhält man durch Umsetzung von 1,8g 2-Methyl-4-ethyl-5-aminosulfonyl-benzoesäuremethylester [erhältlich durch Umsetzung von 3-Aminosulfonyl-4-brom-6-methylbenzoesäuremethylester mit Ethyl-zinkchlorid] mit 1,5 g Guanidin in Methanol das N-Diaminomethylen-2-methyl-4-ethyl-5-aminosulfonyl-benzamid.

Analog erhält man durch Umsetzung von Guanidin
mit 2-Ethyl-4-isopropyl-5-aminosulfonyl-benzoesäuremethylester das N-Diaminomethylen-2-ethyl-4-isopropyl-5-aminosulfonyl-benzamid;
mit 2-Propyl-4-methyl-5-aminosulfonyl-benzoesäuremethylester das N-Diaminomethylen-2-propyl-4-methyl-5-aminosulfonyl-benzamid;
mit 2-Isopropyl-4-methyl-5-aminosulfonyl-benzoesäuremethylester das N-Diaminomethylen-2-isopropyl-4-methyl-5-aminosulfonyl-benzamid;
mit 2-Butyl-4-methyl-5-aminosulfonyl-benzoesäuremethylester das N-Diaminomethylen-2-butyl-4-methyl-5-aminosulfonyl-benzamid;
mit 2-But-2-yl-4-methyl-5-aminosulfonyl-benzoesäuremethylester das N-Diaminomethylen-2-but-2-yl-4-methyl-5-aminosulfonyl-benzamid;
mit 2-Ethyl-4-propyl-5-aminosulfonyl-benzoesäuremethylester das N-Diaminomethylen-2-ethyl-4-propyl-5-aminosulfonyl-benzamid;
mit 2-Propyl-4-ethyl-5-aminosulfonyl-benzoesäuremethylester das N-Diaminomethylen-2-propyl-4-ethyl-5-aminosulfonyl-benzamid;
mit 2-Isopropyl-4-ethyl-5-aminosulfonyl-benzoesäuremethylester das N-Diaminomethylen-2-isopropyl-4-ethyl-5-aminosulfonyl-benzamid;
mit 2-Butyl-4-ethyl-5-aminosulfonyl-benzoesäuremethylester das N-Diaminomethylen-2-butyl-4-ethyl-5-aminosulfonyl-benzamid;
mit 2-(2-Butyl)-4-ethyl-5-aminosulfonyl-benzoesäurernethylester das N-Diaminomethylen-2-but-2-yl-4-ethyl-5-aminosulfonyl-benzamid;
mit 2-Ethyl-4-methyl-5-aminosulfonyl-benzoesäuremethylester das N-Diaminomethylen-2-ethyl-4-methyl-5-aminosulfonyl-benzamid;
mit 2-Chlor-4-methyl-5-aminosulfonyl-benzoesäuremethylester das N-Diaminomethylen-2-chlor-4-methyl-5-aminosulfonyl-benzamid;
mit 2-Brom-4-methyl-5-aminosulfonyl-benzoesäuremethylester das N-Diaminomethylen-2-brom-4-methyl-5-aminosulfonyl-benzamid;
mit 2-Fluormethyl-4-methyl-5-aminosulfonyl-benzoesäuremethylester das N-Diaminomethylen-2-fluormethyl-4-methyl-5-aminosulfonyl-benzamid;
mit 2-Trifluormethyl-4-methyl-5-aminosulfonyl-benzoesäuremethylester das N-Diaminomethylen-2-trifluormethyl-4-methyl-5-aminosulfonyl-benzamid;
mit 2-Pentafluorethyl-4-methyl-5-aminosulfonyl-benzoesäuremethylester das N-Diaminomethylen-2-pentafluorethyl-4-methyl-5-aminosulfonyl-benzamid;
mit 2-Methoxy-4-methyl-5-aminosulfonyl-benzoesäuremethylester das N-Diaminomethylen-2-methoxy-4-methyl-5-aminosulfonyl-benzamid;
mit 2-Cyan-4-methyl-5-aminosulfonyl-benzoesäuremethylester das N-Diaminomethylen-2-cyan-4-methyl-5-aminosulfonyl-benzamid;
mit 2-Nitro-4-methyl-5-aminosulfonyl-benzoesäuremethylester das N-Diaminomethylen-2-nitro-4-methyl-5-aminosulfonyl-benzamid;
mit 2-Ethinyl-4-methyl-5-aminosulfonyl-benzoesäuremethylester das N-Diaminomethylen-2-ethinyl-4-methyl-5-aminosulfonyl-benzamid.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoff enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Alkyl-benzoylguanidine der Formel I 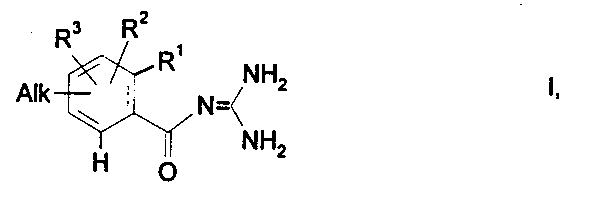 worin
R¹ A, CF₃, CH₂F, CHF₂, C₂F₅, CN, NO₂, Hal, C≡CH oder -X-R⁴,
R² und R³ jeweils unabhängig voneinander H, Hal, A, -X-R⁴, CN, NO₂, CF₃, CH₂F, CHF₂, C₂F₅, CH₂CF₃, -SOₙ-R⁶, -SO₂NR⁴R⁵, Ph, OPh oder Het,
R⁴ H, A, Cycloalkyl mit 5 bis 7 C-Atomen, Cycloalkylmethyl mit 6 bis 8 C-Atomen, CF₃, CH₂F, CHF₂, CH₂CF₃, Ph oder -CH₂-Ph,
R⁵ H oder A oder aber
R⁴ und R⁵ zusammen auch Alkylen mit 4 bis 5 C-Atomen, wobei eine CH₂-Gruppe auch durch O, S, NH, N-A oder N-CH₂-Ph ersetzt sein kann,
R⁶ A oder Ph,
Alk einen geradkettigen oder verzweigten C₁-C₈-Alkyl-Rest, C₃-C₈-Cycloalkyl, weiches unsubstituiert oder durch A ein-, zwei- oder dreifach substituiert sein kann, -CR⁷=CHR^{7'} oder -C≡CR⁷,
R⁷ und R^{7'} jeweils unabhängig voneinander H, A, Ph oder Het,
Het einen ein- oder zweikernigen gesättigten, ungesättigten oder aromatischen Heterocyclus mit 1 bis 4 N-, O-, und/oder S-Atomen, über N oder C gebunden, der unsubstituiert oder ein-, zwei- oder dreifach durch Hal, CF₃, A, -X-R⁴, CN, NO₂ und/oder Carbonylsauerstoff substituiert sein kann.
A Alkyl mit 1 bis 6 C-Atomen,
Hal F, Cl, Br oder I,
X O oder S,
Ph unsubstituiertes oder ein-, zwei- oder dreifach durch A, OA, NR⁴R⁵, F, Cl, Br, I oder CF₃ substituiertes Phenyl und
n 1 oder 2,
bedeuten,
sowie deren physiologisch unbedenkliche Salze.

2. (a) Diaminomethylen-2-methyl-4-isopropyl-5-methylsulfonyl-benzamid;
(b) N-Diaminomethylen-2,4-dimethyl-5-methylsulfonyl-benzamid;
(c) N-Diaminomethylen-2-brom-4-methyl-5-methylsulfonyl-benzamid;
(d) N-Diaminomethylen-2-ethyl-4-isopropyl-5-methylsulfonyl-benzamid;
(e) N-Diaminomethylen-2-ethyl-4-cyclopentyl-5-methylsulfonyl-benzamid;
(f) N-Diaminomethylen-2-methyl-3-methylsulfonyl-4-cyclohexyl-benzamid;
(g) N-Diaminomethylen-2-ethyl-3-methylsulfonyl-4-cyclopentyl-benzamid
gemäß Anspruch 1, sowie deren physiologisch unbedenkliche Salze.

3. Verfahren zur Herstellung von Alkylbenzoylguanidin-Derivaten der Formel I gemäß Anspruch 1 sowie von deren Salzen, dadurch gekennzeichnet, daß man eine Verbindung der Formel II 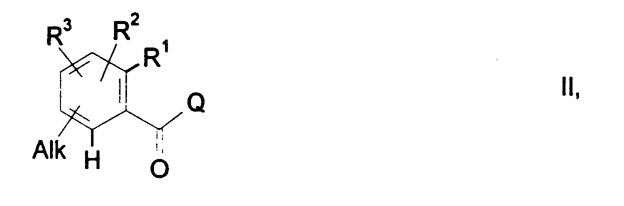 worin Alk, R¹, R² und R³ die zuvor angegebenen Bedeutungen haben
und
Q Cl, Br, OA, O-CO-A, O-CO-Ph, OH oder eine andere reaktionsfähige veresterte OH-Gruppe bzw. leicht nucleophil substituierbare Abgangsgruppe bedeutet,
mit Guanidin umsetzt, oder daß man ein Benzoylguanidin der Formel III 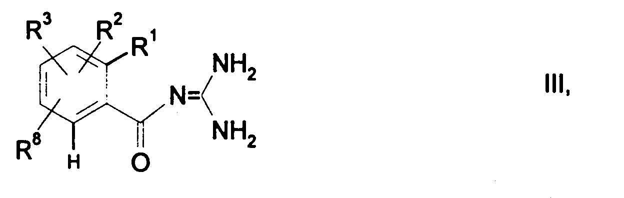 worin R¹, R² und R³ die zuvor angegebenen Bedeutungen besitzen, und
R⁸ F, Cl, Br, I oder H bedeutet,
mit einer Kohlenwasserstoffverbindung der Formel IV
Alk-R^{8'} IV,
worin Alk die angegebene Bedeutung besitzt und
R^{8'} H, Cl, Br oder I bedeutet,
in Gegenwart eines Katalysators, nach vorheriger Metallierung bzw. Transmetallierung, umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man eine erhaltene Base der Formel I durch Behandeln mit einer Säure in eines ihrer Salze umwandelt.

4. Verfahren zur Herstellung pharmazeutischer Zubereitungen, dadurch gekennzeichnet, daß an eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

7. Verwendung von Verbindungen der Formel I nach Anspruch 1 zur Herstellung von Arzneimitteln zur Behandlung von Arrhythmien, Angina pectoris, Infarkten sowie zur präventiven Behandlung der genannten Indikationen.

## Claims

1. Alkylbenzoylguanidines of the formula I 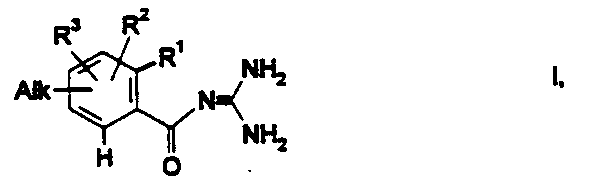 in which
R¹ is A, CF₃, CH₂F, CHF₂, C₂F₅, CN, NO₂, Hal, C≡CH or -X-R⁴,
R² and R³ in each case independently of one another are H, Hal, A, -X-R⁴, CN, NO₂, CF₃, CH₂F, CHF₂, C₂F₅, CH₂CF₃, -SOₙ-R⁶, -SO₂NR⁴R⁵, Ph, OPh or Het,
R⁴ is H, A, cycloalkyl having 5 to 7 C atoms, cycloalkylmethyl having 6 to 8 C atoms, CF₃, CH₂F, CHF₂, CH₂CF₃, Ph or -CH₂₋Ph,
R⁵ is H or A or else
R⁴ and R⁵ together are also alkylene having 4 to 5 C atoms, where one CH₂ group can also be replaced by O, S, NH, N-A or N-CH₂-Ph,
R⁶ is A or Ph,
Alk is a straight-chain or branched C₁-C₈-alkyl radical, C₃-C₈-cycloalkyl which can be unsubsituted or mono-, di- or trisubstituted by A, or is -CR⁷=CHR^{7'} or -C≡CR⁷,
R⁷ and R^{7'} in each case independently of one another are H, A, Ph or Het,
Het is a mono- or polynuclear saturated, unsaturated or aromatic heterocycle having 1 to 4 N, O and/or S atoms, bonded via N or C, which can be unsubstituted or mono-, di- or trisubstituted by Hal, CF₃, A, -X-R⁴, CN, NO₂ and/or carbonyl oxygen,
A is alkyl having 1 to 6 C atoms,
Hal is F, Cl, Br or I,
X is O or S,
Ph is phenyl which is unsubstituted or mono-, di- or trisusbstituted by A, OA, NR⁴R⁵, F, Cl, Br, I or CF₃ and
n is 1 or 2,
and their physiologically tolerable salts.

2. (a) Diaminomethylene-2-methyl-4-isopropyl-5-methylsulfonylbenzamide;
(b) N-diaminomethylene-2,4-dimethyl-5-methylsulfonylbenzamide;
(c) N-diaminomethylene-2-bromo-4-methyl-5-methylsulfonylbenzamide;
(d) N-diaminomethylene-2-ethyl-4-isopropyl-5-methylsulfonylbenzamide;
(e) N-diaminomethylene-2-ethyl-4-cyclopentyl-5-methylsulfonylbenzamide;
(f) N-diaminomethylene-2-methyl-3-methylsulfonyl-4-cyclohexylbenzamide;
(g) N-diaminomethylene-2-ethyl-3-methylsulfonyl-4-cyclopentylbenzamide
according to Claim 1, and their physiologically tolerable salts.

3. Process for the preparation of alkylbenzoylguanidine derivatives of the formula I according to Claim 1, and of their salts, characterized in that a compound of the formula II 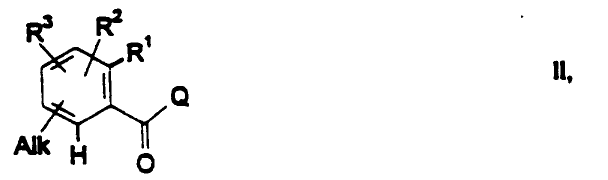 in which Alk, R¹, R² and R³ have the meanings indicated above
and
Q is Cl, Br, OA, O-CO-A, O-CO-Ph, OH or another reactive esterified OH group or easily nucleophilically substitutable leaving group,
is reacted with guanidine,
or in that a benzoylguanidine of the formula III 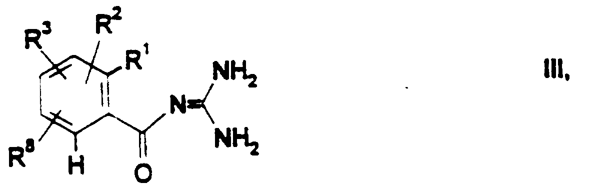 in which R¹, R² and R³ have the meanings indicated above,
and
R⁸ is F, Cl, Br, I or H,
is reacted with a hydrocarbon compound of the formula IV
Alk-R^{8'} IV,
in which Alk has the indicated meaning and
R^{8'} is H, Cl, Br or I,
in the presence of a catalyst, after prior metallation or transmetallation,
or in that a compound otherwise corresponding to the formula I, but which instead of one or more hydrogen atoms contains one or more reducible groups and/or one or more additional C-C and/or C-N bonds, is treated with a reducing agent,
or in that a compound otherwise corresponding to the formula I, but which instead of one or more hydrogen atoms contains one or more solvolysable groups, is treated with a solvolysing agent
and/or in that a base of the formula I obtained is converted into one of its salts by treating with an acid.

4. Process for the production of pharmaceutical preparations, characterized in that a compound of the formula I according to Claim 1 and/or one of its physiologically acceptable salts is brought into a suitable dose form together with at least one solid, liquid or semi-liquid excipient or auxiliary.

5. Pharmaceutical preparation, characterized in that it contains at least one compound of the general formula I according to Claim 1 and/or one of its physiologically acceptable salts.

6. Use of compounds of the formula I according to Claim 1 or of their physiologically acceptable salts for the production of a medicament.

7. Use of compounds of the formula I according to Claim 1 for the production of medicaments for the treatment of arrhythmias, angina pectoris, infarcts, and for the preventive treatment of the indications mentioned.

## Revendications

1. Alkylbenzoylguanidines de formule I 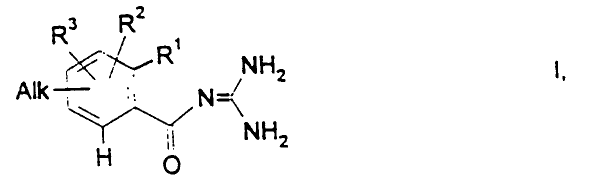 où
R¹ représente les groupes A, CF₃, CH₂F, CHF₂, C₂F₅, CN, NO₂, Hal, C≡CH ou -X-R⁴,
R² et R³ représentent respectivement indépendamment l'un de l'autre les groupes H, Hal, A, -X-R⁴, CN, NO₂, CF₃, CH₂F, CHF₂, C₂F₅, CH₂CF₃, -SOₙ-R⁶, -SO₂NR⁴R⁵, Ph, OPh ou Het,
R⁴ représente les H, A, cycloalkyle de 5 à 7 atomes de C, cycloalkylméthyle ayant 6 à 8 atomes de C, CF₃, CH₂F, CHF₂, CH₂CF₃, Ph ou -CH₂-Ph,
R³ représente H ou A ou aussi
R⁴ et R⁵ représentent ensemble un alkylène avec 4 à 5 atomes de C, où un groupe CH₂ peut aussi être remplacé par O, S, NH, N-A ou N-CH₂-Ph,
R⁶ représente A ou Ph,
Alk représente un reste alkyle C₁-C₈ linéaire ou ramifié, cycloalkyle C₃-C₈, qui peut être non substitue ou être substitué une fois, deux fois ou trois fois par A, -CR⁷=CHR^{7'} ou -C≡CR⁷,
R⁷ et R^{7'} représentent respectivement indépendamment l'un de l'autre les H, A, Ph ou Het,
Het représente un hétérocycle à un ou deux noyaux saturé, non saturé ou aromatique, avec 1 à 4 atomes de N, O et/ou S, liés par l'intermédiaire de N ou C, qui peut être non substitué ou substitué une fois, deux fois, ou trois fois par Hal, CF₃, A, -X-R⁴, CN, NO₂, et/ou un oxygène carbonyle,
A représente un alkyle ayant 1 à 6 atomes de C,
Hal représente F, Cl, Br ou I,
X représente O ou S,
Ph représente un groupe phényle non substitué ou substitué une fois, deux fois ou trois fois par A, OA, NR⁴R⁵, F, Cl,Br, I ou CF₃, et
n représente 1 ou 2,
ainsi que leurs sels physiologiquement non nuisibles.

2. (a) diaminométhylène-2-méthyl-4-isopropyl-5-méthylsulfonylbenzamide ;
(b) N-diaminométhylène-2,4-diméthyl-5-méthyl-sulfonylbenzamide ;
(c) N-diaminométhylène-2-bromo-4-méthyl-5-méthylsulfonylbenzamide ;
(d) N-diaminométhylène-2-éthyl-4-isopropyl-5-méthylsulfonylbenzamide ;
(e) N-diaminométhylène-2-éthyl-4-cyclopentyl-5-méthylsulfonylbenzamide ;
(f) N-diaminométhylène-2-méthyl-3-méthylsulfonyl-4-cyclohexylbenzamide ;
(g) N-diaminométhylène-2-éthyl-3-méthylsulfonyl-4-cyclopentylbenzamide,
selon la revendication 1, ainsi que leurs sels physiologiquement non nuisibles.

3. Procédé de préparation de dérivés d'alkylbenzoylguanidine de formule I selon la revendication 1, ainsi que de leurs sels, caractérisé en ce que l'on fait réagir un composé de formule II 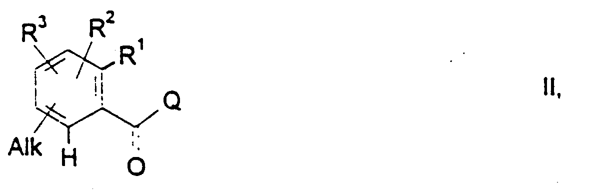 où Alk,R¹,R² et R³ ont les significations données ci-dessus, et
Q représente les Cl, Br,OA,0-CO-A,O-CO-Ph, OH ou un autre groupe OH estérifié réactif, ou un groupe éliminable facilement par réaction nucléophile,
avec la guanidine,
ou en ce que l'on fait réagir une benzoylguanidine de formule III 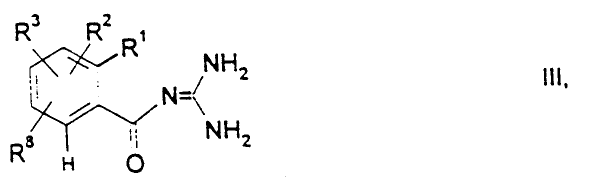 où R¹, R² et R³ ont les significations données ci-dessus, et
R³ représente les F, Cl, Br, I ou H,
avec un composé hydrocarboné de formule IV
Alk-R^{8'} IV
où Alk a la signification déjà donnée et
R^{8'} représente les H, Cl, Br ou I,
en présence d'un catalyseur, après métallisation ou transmétallisation préalable,
ou en ce que l'on traite avec un agent réducteur un composé qui répond sinon à la formule I, qui contient cependant à la place d'un ou plusieurs atomes d'hydrogène, un ou plusieurs groupes réductibles et/ou une ou plusieurs liaisons supplémentaires C-C et/ou C-N,
ou en ce que l'on traite avec un agent de solvolyse un composé qui correspond sinon à la formule I, qui contient cependant à la place d'un ou plusieurs atomes d'hydrogène un ou plusieurs groupes solvolysables,
et/ou en ce que l'on transforme une base obtenue de formule I en l'un de ses sels par traitement avec un acide.

4. Procédé de production de préparations pharmaceutiques, caractérisé en ce que l'on met sous forme posologique appropriée un composé de formule I selon la revendication 1, et/ou un de ses sels physiologiquement non nuisibles en présence d'au moins un véhicule ou un adjuvant solide, liquide ou semiliquide.

5. Préparation pharmaceutique caractérisée en ce qu'elle contient une certaine quantité d'au moins un composé de formule générale I selon la revendication 1 et/ou un de ses sels pnysiologiquement non nuisibles.

6. Utilisation de composés de formule I selon la revendication 1 ou de leurs sels physiologiquement non nuisibles pour produire un médicament.

7. Utilisation des composés de formule I selon la revendication 1 pour produire des médicaments afin de traiter les arythmies, l'angine de poitrine, les infarctus, ainsi que pour traiter préventivement les risques indiqués.
